# EUROPÄISCHE PATENTSCHRIFT

(11) **EP 2 563 223 B1**
(45) Veröffentlichungstag und Bekanntmachung des Hinweises auf die Patenterteilung: **25.06.2014**
(21) Anmeldenummer: 11717947.3
(22) Anmeldetag: 26.04.2011
(51) Int. Cl.: A61B 5/151

(54) **STECHHILFE MIT AUTOMATISCHER AUSLÖSUNG**
LANCING DEVICE WITH AUTOMATIC DISENGAGEMENT
AIDE À LA PLANTATION DOTÉE D'UN DÉCLENCHEMENT AUTOMATIQUE

(30) Priorität: 30.04.2010 EP 10004578
(43) Veröffentlichungstag der Anmeldung: 06.03.2013
(73) Patentinhaber: Roche Diagnostics GmbH, 68298 Mannheim (DE); F.Hoffmann-La Roche AG, 4070 Basel (CH)
(72) Erfinder: KEIL, Michael, 67117 Limburgerhof (DE)
(86) Internationale Anmeldenummer: PCT/EP2011/002083
(87) Internationale Veröffentlichungsnummer: WO 2011/134639

(56) Entgegenhaltungen:
- EP-A1- 1 034 740
- EP-A1- 1 384 438
- EP-A1- 2 033 577
- EP-A2- 1 504 718
- WO-A1-02/36010
- WO-A2-2008/145625
- US-A1- 2003 050 656
- US-B2- 6 986 777

## Beschreibung

Die vorliegende Erfindung betrifft eine Stechhilfe zur Entnahme von Körperflüssigkeiten für diagnostische Zwecke mit gekoppeltem Spann- und Auslösemechanismus, wobei der Auslösemechanismus derart mit dem Spannmechanismus mechanisch gekoppelt ist, dass bei Betätigung des Spannmechanismus der Stechvorgang durch kontinuierliche Fortsetzung der Spannbewegung automatisch ausgelöst wird und wobei der derart ausgelöste Stechvorgang durch eine Lanzette ausgeführt wird.

Bei verschiedenen Krankheiten ist es notwendig, Körperflüssigkeiten, insbesondere das menschliche Blut, hinsichtlich eines darin enthaltenen Analyten zu untersuchen. In vielen Fällen reicht es dabei aus, durch Erzeugung einer kleinen Stichwunde dem Körper eine geringe Menge der gewünschten Körperflüssigkeit, speziell Blut in Form eines Blutstropfens, zu entnehmen. Ein besonders wichtiger derartiger Fall ist der Diabetes, bei dem das Blut in regelmäßigen Abständen auf den Glukosegehalt untersucht werden muss. Weitere Blutuntersuchungen können zum Beispiel bezüglich der Gerinnungsparameter, Triglyzeride, HbAlc oder Laktat vorgenommen werden. Zur Erzeugung der erforderlichen Stichwunden werden üblicherweise Blutlanzettenvorrichtungen verwendet, die aus einem Stechgerät und hierzu angepassten auswechselbaren Lanzetten bestehen. In dem Gehäuse des Stechgerätes befindet sich ein Lanzettenhalter, in dem jeweils eine Lanzette auswechselbar eingesetzt werden kann. Beim Einstichvorgang wird der Lanzettenhalter von einem ebenfalls in dem Stechgerät integrierten Lanzettenantrieb schnell in eine Einstichrichtung bewegt, bis die Lanzettespitze aus einer vom vorderen Ende des Stechgerätes vorgesehen Austrittsöffnung austritt und eine kleine Stichwunde in dem Körperteil, gegen das das vordere Ende gedrückt wird, erzeugt. Danach wird der Lanzettenhalter mit der Lanzette entgegen der Einstichrichtung zurückbewegt.

Im Laufe der Zeit haben sich kleine handliche Entnahmegeräte, sogenannte Stechhilfen, etabliert, die vom Benutzer einfach und zuverlässig bedient werden können und einen möglichst schmerzarmen Einstich in ein Körperteil zu ermöglichen. Zur Vermeidung von Infektionen, insbesondere im Krankenhaus, sind die Lanzetten zur einmaligen Verwendung vorgesehene disposible Elemente. Nach der einmaligen Verwendung einer Lanzette wird die Lanzette nach dem Stechvorgang entnommen oder vom Gerät ausgeworfen und entsorgt.

Neben der Verwendung der Blutlanzettenvorrichtung durch medizinisches Personal werden darüber hinaus Stechhilfen auch von Laien im sogenannten Home-Monitoring-Bereich eingesetzt. Dies gilt insbesondere für die Therapiekontrolle von Diabetikern. So wurde bei der Behandlung von Diabetikern festgestellt, dass schwerwiegende, mit dem Diabetes verbundene Schäden, wie zum Beispiel Erblinden, entscheidend vermindert werden können, wenn die Glukosekonzentration im Blut des Diabetikers häufig, bis zu fünf mal täglich, bestimmt wird und aufgrund dieser Messungen die Insulininjektion exakt eingestellt werden kann. Um derart häufige Messungen zu realisieren, werden Stechhilfen im Rahmen des Home-Monitoring-Bereiches eingesetzt, sodass der Diabetiker selbst eine Blutuntersuchung vornehmen kann. Die Anforderungen, die sich hierdurch an eine Blutlanzettenvorrichtung ergeben, sind neben einer einfachen Handhabung beim Auslösen des Stechvorgangs sowie eines schmerzarmen Einstiches auch ein einfaches Handling beim Einsetzen neuer Lanzetten sowie ein sicheres Verwerfen von bereits gebrauchten Lanzetten. Ein Wechsel der Lanzetten sollte einerseits möglichst einfach sein, andererseits aber auch ein Maximum an Sicherheit gegen ungewollte Verletzungen des Anwenders sowie Dritter gewährleisten. Hierbei ist es im Home-Monitoring-Bereich denkbar, dass eine einmal eingelegte Lanzette durch den selben Benutzer zwar mehrmals zum Stechen verwendet wird; jedoch sollte auch hier, nachdem der Benutzer sich entschieden hat, die Lanzette zu verwerfen, eine versehentliche Wiederverwendung einer bereits ausgeworfenen Lanzette verhindert werden. Darüber hinaus sollte ein Schutz vor ausgeworfenen Lanzetten insbesondere für Dritte, beispielsweise bei der Müllbeseitigung, gewährleistet sein.

Aus vorstehend genannten Gründen besteht daher stetiger Bedarf an Vorrichtungen, die es dem Anwender im Home-Monitoring -Bereich auf möglichst angenehme, unkomplizierte und insbesondere möglichst schmerzarme Weise ermöglichen, seinen Blutglucosespiegel oder auch die Konzentration anderer Analyten in anderen Körperflüssigkeiten durch möglichst häufige Messungen zu überwachen.

Die EP 0 668 049 A1 offenbart eine automatisch spannende Lanzetteneinrichtung umfassend einen Auslöseknopf, der mit einer Auslöseknopffeder und einer Antriebsfeder beaufschlagt ist, die den Auslöseknopf mit einem Druckstempel verbindet. Beim Auslösen ist die Auslöseknopffeder komprimiert, und sobald der Benutzer den Auslöseknopf freigibt, bringt die Auslöseknopffeder den Druckstempel und den Auslöseknopf unter der Vorspannkraft der Auslöseknopffeder in die gespannte Position zurück. Die offenbarte Lanzetteneinrichtung spannt sich automatisch und ist daher ständig gespannt und auslösebereit. Spannen und Auslösen der Lanzetteneinrichtung erfolgen demnach in zwei separat vorzunehmenden Betätigungen des Auslöseknopfes.

Die US 2008/0195132 A1 offenbart spezielle Stechhilfen mit je einer Spann- und einer Antriebsfeder, wobei die Antriebsfeder mit einem Haltelement verbunden ist, das zur Aufnahme der Lanzette dient. Die Stechhilfen weisen im Rahmen einer Ausführungsform ein automatisches Spann- und Auslösesystem auf, bei dem die Stechhilfe zunächst durch Betätigung des Spannknopfes gespannt wird und anschließend durch Loslassen des Auslöseknopfes, d.h. durch Entspannen der Spannfeder automatisch ausgelöst wird. Durch Expansion der gespannten Antriebsfeder wird das Halteelement und die damit verbundene Lanzette in Einstichrichtung angetrieben und durch Entspannung derselben wieder in das Gehäuse zurückgezogen.

Gegenstand der WO 03/073936 A2 ist eine Vorrichtung zur Punktion der Haut eines Patienten zur einmaligen Benutzung, die ein Druckelement aufweist, mit dem sowohl eine Antriebsfeder gespannt werden kann als nach mechanischer Überbeanspruchung von internen Halteelementen eine Punktionseinheit zur Ausführung einer Punktionsbewegung freigegeben werden kann. Dazu wird die Vorrichtung auf die zu punktierende Hautpartie aufgesetzt und mittels des Druckelementes zusammengedrückt. Nach Ausführung der Einstichbewegung wird die frei fliegende Punktionseinheit durch eine Rückholfeder in eine Position innerhalb der Vorrichtung zurückgezogen.

Die WO 02/05872 A2 offenbart eine Einweg-Stechhilfe, die sich durch eine einzelne, in lediglich eine Richtung geführte Betätigung eines Auslösers sowohl Spannen als auch Auslösen lässt. Dabei wird eine Lanzette mittels einer gespannten Feder aus einer sterilen Umgebung innerhalb der Vorrichtung in Einstichrichtung angetrieben und nach dem Einstich wieder in das Gehäuse zurückgezogen. Die Vorrichtung ermöglicht weder die individuelle Anpassung der Einstichtiefe noch der Antriebskraft bzw. - geschwindigkeit.

In der US 6,986,777 wird eine automatische Stechhilfe beschrieben, die ebenfalls einen einzigen Auslöseknopf sowohl zum Spannen als auch zum Auslösen aufweist. Die Stechhilfe weist ferner einen Mechanismus auf, bei dem ein Führungsbolzen in einer Führung bewegt wird, wobei die Führung einen umlaufenden Teil sowie einen längslaufenden Abschnitt aufweist. Beim Betätigen des Auslöseknopfes wird die Stechhilfe gespannt und der Führungsbolzen in der Führung verschoben, wobei beim Erreichen des längslaufenden Abschnittes der Führung die automatisch eine Stechbewegung ausgelöst wird. Dabei wird ein zunächst nicht mit der Lanzette in Eingriff befindlicher Stößel in Richtung Lanzette beschleunigt und durch Auftreffen des Stößels auf die Lanzette die Einstichbewegung durchgeführt.

Die EP 1 384 438 A1 offenbart ein Blutentnahmesystem zur Entnahme von Blut für Diagnosezwecke, umfassend ein Gehäuse, eine Lanzettenführung und einen Lanzettenantrieb mit einer Antriebsfeder. Zu dem Lanzettenantrieb gehört eine Spanneinrichtung, um die Antriebsfeder zu spannen, ein von der Antriebsfeder angetriebener Antriebsrotor und ein abtriebsseitiger Kopplungsmechanismus, durch den die Drehbewegung des Antriebsrotors in die Einstichbewegung umgesetzt wird. Dabei wird auch die Möglichkeit erwähnt, den Lanzettenantrieb mit einer selbstauslösenden Hemmung zu versehen.

Ausgehend von diesem Stand der Technik bestand die Aufgabe der vorliegenden Erfindung einer Stechhilfe, die sich
- mit möglichst geringem Kraftaufwand,
- in möglichst einfacher und intuitiver Weise, insbesondere mit einer möglichst geringen Anzahl von Bedienschritten,
- unter Erzeugung eines möglichst geringen Einstichschmerzes
bedienen lässt und sich dabei durch eine gute Handhabbarkeit, insbesondere durch möglichst geringe äußere Abmessungen sowie durch eine möglichst geringe Anzahl zusätzlicher Bauteile und durch eine dadurch wirtschaftliche Herstellbarkeit auszeichnet. Darüber hinaus soll sie dazu beitragen, beim Anwender die Hemmschwelle zur Erzeugung der Einstichwunde herabzusetzen.

Die Aufgabe wurde erfindungsgemäß gelöst durch die Bereitstellung eines Systems zur Entnahme von Körperflüssigkeiten für Diagnosezwecke, umfassend
- ein Entnahmegerät,
- ein Gehäuse mit einer Austrittsöffnung für die Lanzettenspitze,
- einen in dem Gehäuse entlang eines vorbestimmten Einstichweges beweglichen Lanzettenhalter zur auswechselbaren Halterung der Lanzette,
- ein Magazin mit einer Mehrzahl von auf das Entnahmegerät abgestimmten Lanzetten mit einem Lanzettenkörper und einer Lanzettenspitze, die nacheinander an den Lanzettenhalter angekoppelt werden können, wobei die vorgesehenen Lanzetten mit je einem Sterilschutz versehen sind, der die Sterilität von noch unbenutzten Lanzetten bis zum Einstich gewährleistet und der aus einem elastomeren Material besteht und beim Einstich durch die zu schützende Lanzettenspitze durchstochen oder abgestreift wird,
- eine Lanzettenführung zur Führung des Lanzettenhalters auf dem vorbestimmten Einstichweg nach Auslösen der Einstichbewegung,
- einen Lanzettenantrieb mit einem elastischen Antriebselement, der sich durch Spannen aus einem ungespannten in einen gespannten Zustand überführen lässt und durch den nach dem Auslösen die Entspannungsbewegung des gespannten elastischen Antriebselementes in eine Einstichbewegung umgesetzt wird, bei der die vom Lanzettenhalter gehaltene Lanzette entlang des vorbestimmten Einstichweges in Einstichrichtung bewegt wird, bis die Lanzettenspitze zumindest teilweise aus der Austrittsöffnung austritt und durch den der Lanzettenhalter in eine Position zurückgeführt wird, bei der die Spitze der Lanzette sich in dem Gehäuse befindet, und
- eine kombinierte Spann- und Auslöseeinrichtung mit einem von der Außenseite des Gehäuses zugänglichen Betätigungselement, das einen Ausgangszustand und einen betätigten Zustand aufweist, wobei die kombinierte Spann- und Auslöseeinrichtung eine Arretiervorrichtung aufweist, die mit dem Betätigungselement und dem Lanzettenantrieb derart mechanisch gekoppelt ist, dass der Lanzettenantrieb bei Verschieben des Betätigungselementes entlang des Betätigungsweges zunächst gespannt und dann ausgelöst wird, wobei die Auslösung des Lanzettenantriebs bei Erreichen eines bestimmten Punktes entlang des Betätigungsweges freigegeben wird.

Das erfindungsgemäße System eignet sich zur Entnahme von Körperflüssigkeiten wie beispielsweise Blut oder interstitieller Flüssigkeit, bevorzugt zur Entnahme von Blut für Diagnosezwecke. Da für Diagnosezwecke, in der Regel lediglich sehr geringe Mengen an zu untersuchender Körperflüssigkeit, vorzugsweise Blut, benötigt werden, dient das erfindungsgemäße System zur Entnahme geringer Mengen der jeweiligen Körperflüssigkeit, vorzugsweise von Blut, wobei die zu entnehmende Menge üblicherweise in einer Menge von etwa 0,5 bis etwa 5 µl, oft in einer Menge von etwa 1 bis etwa 3 µl beträgt.

Zur Entnahme der genannten geringen Mengen der gewählten Körperflüssigkeit, vorzugsweise Blut, wird üblicherweise unter Einsatz des erfindungsgemäßen Systems eine Inzision in einer zuvor ausgewählten Hautpartie, beispielsweise an einer Fingerkuppe oder am Ohrläppchen herbeigeführt. Die gewünschte Inzision wird dabei üblicherweise durch einen möglichst schnellen und kurzen Einstich der vom erfindungsgemäßen System umfassten Lanzette in die gewählte Hautpartie herbeigeführt. Dadurch kann die gewünschte geringe Menge der jeweiligen Körperflüssigkeit aus der gewählten Hautpartie austreten.

Vorzugsweise handelt es sich bei dem System der vorliegenden Erfindung um ein wieder verwendbares System zur Entnahme von Körperflüssigkeiten, vorzugsweise von Blut, d.h. um ein solches System, das nicht nur zum einmaligen Gebrauch bestimmt ist. Demzufolge kann das erfindungsgemäße System, genauer das vom System umfasste Entnahmegerät, ein- oder mehrmals hintereinander eingesetzt werden, vorzugsweise nach Austausch der jeweils eingesetzten benutzten Lanzette durch eine unbenutzte Lanzette.

Das erfindungsgemäße System umfasst ein Entnahmegerät und eine Mehrzahl von auf das Entnahmegerät abgestimmte Lanzette mit einem Lanzettenkörper und einer Lanzetten-spitze. Wie vorstehend bereits ausgeführt eignet sich das Entnahmegerät sowohl zur einmaligen als auch zur mehrmaligen Benutzung, bevorzugt jedoch zu mehrmaligen Nutzung. Im Rahmen einer bevorzugten Ausführungsform kann das erfindungsgemäße System mehrfach genutzt werden, wahlweise unter wiederholter Nutzung einer bereits benutzten Lanzette oder bevorzugt nach Austausch einer Bereits benutzten Lanzette durch eine noch unbenutzte Lanzette. Dabei ist unter einer benutzten Lanzette eine solche zu verstehen, mit der bereits eine oder mehrere Einstiche durchgeführt wurden.

Die vom erfindungsgemäßen System umfassten Lanzetten weisen eine Lanzettenspitze und einen Lanzettenkörper auf, die sowohl einstückig als auch mehrstückig ausgestaltet sein können. Im Falle der mehrstückigen Ausgestaltung können Lanzettenkörper und Lanzettenspitze sowohl aus dem gleichen als auch bevorzugt aus unterschiedlichen Materialien gefertigt sein. Als geeignete Materialien für die Lanzettenspitze seien Metalle wie beispielsweise Edelstahl oder speziell Federstahl genannt. Vorzugsweise handelt es sich bei dem im Rahmen des erfindungsgemäßen Systems einzusetzenden Lanzetten um solche, die eine aus Edelstahl gefertigte Lanzettenspitze umfassen, die mit einem vorzugsweise aus einem geeigneten Kunststoff, besonders bevorzugt aus Acrylnitril-Butadien-Styrol gefertigten Lanzettenkörper verbunden sind.

Weiterhin weisen die im Rahmen des erfindungsgemäßen Systems einzusetzenden Lanzetten vorzugsweise einen Schutzkörper auf, der die zur Erzeugung der Hautinzision dienende Lanzettenspitze so umgibt, dass eine Kontamination der Lanzettenspitze mit Verunreinigungen oder Keimen vermieden werden kann und die Sterilität einer noch unbenutzten Lanzette gewährleistet werden kann. Derartige, auch als Sterilschutz zu bezeichnende Schutzkörper sind dem Fachmann bekannt und beispielsweise in der EP 1 263 320 A1 beschrieben. Sie werden üblicherweise aus einem geeigneten Kunststoff gefertigt und können sowohl vor der Benutzung der Lanzette oder auch während der Benutzung, d.h. während des Einstiches von der Lanzettenspitze entfernt werden.

Das erfindungsgemäße System, konkret das vom genannten System umfasste Entnahmegerät weist ein Gehäuse mit einer Austrittsöffnung für die Lanzettenspitze auf. Das Gehäuse weist dabei vorzugsweise eine langgestreckte Form auf, die vorzugsweise eine Hauptachse (A) aufweist. Dabei befindet sich die genannte Austrittsöffnung für die Lanzettenspitze an einem Ende des langgestreckten Gehäuses. Dieses Ende wird im Rahmen der vorliegenden Erfindung als proximales Ende des Gehäuses bezeichnet.

Das erfindungsgemäße System umfasst weiterhin einen in dem Gehäuse entlang eines vorbestimmten, üblicherweise geradlinigen Einstichweges beweglichen Lanzettenhalter zur auswechselbaren Halterung der Lanzette. Vorzugsweise ist dabei der vorbestimmte Einstichweg parallel zur Richtung der Hauptachse (A) des Gehäuses ausgerichtet. Der erfindungsgemäß vorgesehene Lanzettenhalter dient zur Aufnahme der Lanzette und ist vorteilhaft so ausgestaltet, dass er die Lanzette während der Verschiebung entlang des Einstichweges zur Herbeiführung der vorstehend genannten Hautinzision festhält, d.h. sich mit der Lanzette in dauerhafter aber lösbarer mechanischer Kopplung befindet. Unter dem Begriff der auswechselbaren Halterung ist demgemäß im Rahmen der vorliegenden Erfindung zu verstehen, dass der Lanzettenhalter zumindest im Verlauf des Einstichvorganges so mit der Lanzette verknüpft ist, dass die mechanische Kopplung zwischen Lanzette, bevorzugt zwischen Lanzettenkörper und Lanzettenhalter ununterbrochen aufrecht erhalten wird und nach Abschluss des Einstichvorgangs gelöst oder beibehalten werden kann. Auf diese Weise kann eine benutzte Lanzette nach Durchführung eines Einstichvorganges wahlweise weiterbenutzt oder entfernt und durch eine weitere, vorzugsweise unbenutzte Lanzette ersetzt werden.

Die mechanische Kopplung zwischen Lanzette und Lanzettenhalter kann beispielsweise durch Presssitz oder durch Verklemmen oder Verrasten realisiert sein. Ein im Rahmen des erfindungsgemäßen Systems geeigneter Lanzettenhalter ist beispielsweise in der EP 0 565 970 A1 beschrieben. Demgemäß kann der Lanzettenkörper beispielsweise von dem Lanzettenhalter formschlüssig umgeben sein. Eine formschlüssige Kopplung zwischen Lanzettenkörper und Lanzettenhalter ist auch möglich, wenn der Lanzettenhalter als Stößel ausgestaltet ist, der eine Haltevorrichtung aufweist durch die eine lösbare formschlüssige Kopplung mit dem Haltebereich einer Lanzette hergestellt wird, wie sie beispielsweise in der WO 02/36010 A1 beschrieben sind, auf die diesbezüglich hiermit Bezug genommen wird. Letztere Ausführung der formschlüssigen Kopplung zwischen einem als Stößel ausgestalteten Lanzettenhalter und einer Lanzette mit einem Haltebereich hat sich als vorteilhaft erwiesen, da mehrere Lanzetten in Form eines Lanzettenmagazins im Rahmen des erfindungsgemäßen Systems eingesetzt werden.

Das Entnahmegerät des erfindungsgemäßen Systems umfasst weiterhin eine Lanzettenführung zur Führung des Lanzettenhalters auf dem vorbestimmten, üblicherweise geradlinigen Einstichweg nach Auslösen der Einstichbewegung. Der Einstichweg verläuft bevorzugt entlang einer zur Hauptachse (A) des Gehäuses parallelen Gerade. Die Lanzettenführung ermöglicht es in vorteilhafter Weise, dass der Lanzettenhalter und somit die darin gehaltene bzw. damit verbundene Lanzette zumindest während des gesamten Einstichvorganges in mechanischer Kopplung mit dem Lanzettenantrieb stehen kann. Dabei ist unter dem Begriff Einstichvorgang im Rahmen der vorliegenden Erfindung der gesamte Bewegungszyklus der Lanzette zu verstehen, bei dem die Lanzette ausgehend von einer im Gehäuse befindlichen Ruheposition zunächst in Richtung der Austrittsöffnung am proximalen Ende des Gehäuses bewegt wird, dort zumindest mit einem Teil der Lanzettenspitze aus dem Gehäuse austritt, am Punkt maximaler Verschiebung die Bewegungsrichtung umkehrt und sich in entgegen gesetzter Richtung zurückbewegt, bis sich die Lanzettenspitze wieder in einer Position innerhalb des Gehäuses befindet, vorzugsweise bis die Lanzette ihre anfängliche Ruheposition wieder erreicht hat. Die Lanzette kann dabei vorteilhaft in dauerhaftem Kontakt mit weiteren Führungselementen wie beispielsweise einer oder mehrerer Führungsschienen stehen. Die genannten Führungselemente bzw. Führungsschienen können beispielsweise auf der Innenseite des Gehäuses des erfindungsgemäßen Systems angebracht sein. Bevorzugt können sie auch Bestandteil eines wie nachstehend näher beschriebenen Lanzettenmagazins sein, Beispielsweise können sie auf der inneren Oberfläche eines Magazingehäuses angebracht sein. Alternativ kann auch das Magazingehäuse selbst so ausgestaltet sein, dass es als Führungselement dient. So können beispielsweise die einzelnen magazinierten Lanzetten in einzelnen Kompartimenten, vorzugsweise in einzelnen Schächten gelagert sein, wobei die jeweiligen Schachtwände als Führungselemente dienen können. Auf diese Weise kann die Bewegung der Lanzette im Rahmen der Einstichbewegung zusätzlich stabilisiert werden und darüber hinaus ein dauerhafter Kontakt zwischen dem Lanzettenantrieb und einer mit dem Lanzettenhalter verbundenen Lanzette erreicht werden, was insbesondere im Hinblick auf eine schmerzarme Herbeiführung der Hautinzision wünschenswert ist.

Das erfindungsgemäße System umfasst weiterhin einen Lanzettenantrieb mit einem elastischen Antriebselement, der sich durch Spannen aus einem ungespannten in einen gespannten Zustand überführen lässt und durch den nach dem Auslösen die Entspannungsbewegung des gespannten elastischen Antriebselementes in eine Einstichbewegung umgesetzt wird, bei der die vom Lanzettenhalter gehaltene Lanzette entlang des vorbestimmten Einstichweges in Einstichrichtung bewegt wird, bis die Lanzettenspitze zumindest teilweise aus der Austrittsöffnung austritt und durch den der Lanzettenhalter vorzugsweise in eine Position zurückgeführt wird, bei der die Spitze der Lanzette sich in dem Gehäuse befindet.

Als im Rahmen der vorliegenden Erfindung geeignete elastische Antriebselemente seien insbesondere Federn genannt, die aus allen, dem Fachmann geeignet erscheinenden elastischen Materialien, insbesondere Kunststoffen und bevorzugt Metallen wie beispielsweise Federstahl 1.4310 gefertigt sein können und die in verschiedenen Ausgestaltungen, beispielsweise als Spiral- oder Blattfedern, bevorzugt als Spiralfedern, eingesetzt werden können. Das Spannen der genannten elastischen Antriebselemente kann in verschiedener Weise erfolgen, beispielsweise durch Dehnung von Spiralfedern entlang deren Rotationsachse oder bevorzugt durch Verdrehung der genannten Spiralfedern um ihre Rotationsachse. Alternativ ist es auch möglich, Blattfedern durch Auslenkung aus der Ruheposition zu spannen.

Aus dem so zu bewerkstelligenden gespannten Zustand des elastische Antriebselementes wird durch Auslösen desselben die Entspannungsbewegung des gespannten elastischen Antriebselementes in eine Einstichbewegung des Lanzettenhalters und somit der darin gehaltenen Lanzette umgesetzt. Im Rahmen dieser Einstichbewegung wird, wie vorstehend erläutert, die vom Lanzettenhalter gehaltene Lanzette entlang des vorbestimmten Einstichweges in Einstichrichtung bewegt, bis die Lanzettenspitze zumindest teilweise aus der Austrittsöffnung austritt und durch den der Lanzettenhalter in eine Position zurückgeführt wird, bei der die Spitze der Lanzette sich in dem Gehäuse, vorzugsweise bis der Lanzettenhalter bzw. die Lanzette sich wieder in der anfänglichen Ruheposition befindet. Dabei hat es sich als vorteilhaft erwiesen, dass sich der Lanzettenhalter und somit auch eine damit verbundene Lanzette vorzugsweise während der gesamten Einstich- und Rückholbewegung einer Lanzette in dauerhafter mechanischer Kopplung mit dem Lanzettenantrieb befindet und dadurch der Lanzettenantrieb sowohl die Einstich- als auch die Rückholbewegung von Lanzettenhalter bzw. Lanzette kontinuierlich antreiben kann. Derartige zwangsführende Antriebe sind dem Fachmann beispielsweise in Form von Kurbelgetrieben, Hebelgetrieben, Kulissen- oder Nockensteuerungen bekannt.

Als im Rahmen der vorliegenden Erfindung besonders vorteilhaft einzusetzender Antrieb hat sich ein solcher erwiesen, der ein Getriebe aufweist, durch welches ein auf der Eingangsseite des Getriebes eingeleitetes Drehmoment in eine Längsverschiebung in Richtung des vorbestimmten Einstichweges umgewandelt wird, wobei die ausgangsseitige Längsverschiebung des Getriebes auf den Lanzettenhalter übertragen wird. Derartige Antriebe sind beispielsweise aus der EP 0 565 970 A1 sowie der EP 1 384 438 A1 bekannt, auf die hiermit diesbezüglich Bezug genommen wird. Der Begriff "Getriebe" wird im Rahmen der vorliegenden Erfindung, wie in den genannten Schriften, im allgemeinen Sinn verstanden, d.h. als eine kinematische Vorrichtung, die zur Kopplung und Umwandlung von Bewegungen dient, wobei im vorliegenden Fall die Bewegung bei der Entspannung des elastischen Antriebselementes, bevorzugt der elastischen Antriebsfeder, in die Bewegung des Lanzettenhalters bzw. der darin auswechselbar gehaltenen Lanzette umgewandelt wird.

Im Rahmen einer bevorzugten Ausführungsform des erfindungsgemäßen Systems weist der Lanzettenantrieb ein Drehschiebergetriebe mit einem drehbaren Antriebsrotor auf, durch welchen ein auf der Eingangsseite des Drehschiebergetriebes eingeleitetes Drehmoment in eine Längsverschiebung in Richtung des vorbestimmten Einstichweges umgewandelt wird, wobei der Antriebsrotor des Drehschiebergetriebes mit dem elastischen Antriebselement gekoppelt ist und die ausgangsseitige Längsverschiebung des Drehschiebergetriebes auf den Lanzettenhalter übertragen wird. Dabei ist es möglich, dass der Antriebsrotor sowohl um eine zu dem vorbestimmten Einstichweg parallele Drehachse als auch um eine zu dem vorbestimmten Einstichweg senkrechte Drehachse drehbar ist. Beispielsweise ist bevorzugt, dass der Antriebsrotor um eine zu dem vorbestimmten Einstichweg, d.h, bevorzugt um eine zur Hauptachse (A) des Gehäuses parallele Drehachse drehbar ist.

Vorzugsweise erfolgt die Übertragung der Drehbewegung des Antriebsrotors in eine zu seiner Drehachse parallele Translationsbewegung mit Hilfe einer Kurvensteuerung, wobei mindestens ein Teil der Einstich- und bevorzugt auch der Rückholbewegung, besonders bevorzugt die gesamte Einstich- und Rückholbewegung, durch eine Relativbewegung des Steuerzapfens in einer die Steuerkurve bildenden Ausnehmung bestimmt wird, bei der der Zapfen eine durch die Ausnehmung gebildete Steuerkurve abfährt. Vorzugsweise weist das Drehschiebergetriebe eine mit dem Antriebsrotor drehbare Ausnehmung auf, in die ein passender Steuerzapfen eingreift, wobei mindestens ein Teil der Einstich- und Rückführbewegung durch eine Relativbewegung zwischen dem Steuerzapfen und der Ausnehmung bestimmt wird, bei der der Steuerzapfen die durch die Ausnehmung gebildete Steuerkurve abfährt. Im Rahmen einer besonders bevorzugten Ausführungsform des erfindungsgemäßen Systems ist die die Steuerkurve bildende und zusammen mit dem Antriebsrotor drehbare Ausnehmung so ausgestaltet, dass der Zapfen diese bei Betätigung des Bedienelementes in einer stetigen, d.h. ununterbrochenen Bewegung vollständig oder zumindest teilweise, vorzugsweise jedoch vollständig abfährt. Im Rahmen einer weiterhin bevorzugten Ausführungsform ist der Antriebsrotor als zylindrische Hülse ausgebildet, innerhalb der ein kolbenförmiges Teil angeordnet ist, das bei der Längsverschiebung in Richtung des Einstichweges mit einer zylindrischen Außenwand in der Hülse gleitet

Im Rahmen einer vorteilhaften Ausgestaltung weist das erfindungsgemäße System einen Lanzettenantrieb mit zwei Rotoren auf, wobei der erste als Spannrotor und der zweite als Antriebsrotor zu bezeichnen ist Die genannten Rotoren sind wiederum bevorzugt durch eine wie vorstehend beschriebene Antriebsfeder miteinander verknüpft und weisen weiter bevorzugt die gleiche Drehachse parallel zur Hauptgeräteachse (A) des erfindungsgemäßen Systems auf. Spann- und Antriebsrotor weisen bei dieser Ausgestaltung die gleiche Drehrichtung auf und drehen nacheinander um jeweils 360°. Aus diesem Grund wird dieser Antrieb auch als "360°-Antrieb" bezeichnet. Eine detaillierte Beschreibung dieses Antriebs ist beispielsweise in der EP 1 034 740 A1 zu finden.

Das erfindungsgemäße System umfasst weiterhin eine kombinierte Spann- und Auslöseeinrichtung mit einem von der Außenseite des Gehäuses zugänglichen Betätigungselement, das einen Ausgangszustand und einen betätigten Zustand aufweist, wobei die kombinierte Spann- und Auslöseeinrichtung eine Arretiervorrichtung aufweist, die mit dem Betätigungselement und dem Lanzettenantrieb derart mechanisch gekoppelt ist, dass der Lanzettenantrieb bei Verschieben des Betätigungselementes entlang des Betätigungsweges, vorzugsweise in Richtung des proximalen Endes des erfindungsgemäßen Systems, zunächst gespannt und dann ausgelöst wird, wobei die Auslösung des Lanzettenantriebs bei Erreichen eines bestimmten Punktes entlang des Betätigungsweges freigegeben wird.

Die kombinierte Spann- und Auslösevorrichtung dient gemäß der vorliegenden Erfindung sowohl zum Spannen als auch zum Auslösen des wie vorstehend beschriebenen Lanzettenantriebs. Sie umfasst ein Betätigungselement, das von der Außenseite des Gehäuses zugänglich ist und das durch Betätigen, d.h. Verschieben von einem Ausgangszustand in einen betätigten Zustand überführt werden kann. Dabei ist im Rahmen einer bevorzugten Ausführungsform das Betätigungselement derart angeordnet, dass es durch das Verschieben zumindest teilweise in das Gehäuse eingeführt wird. Weiterhin bevorzugt wird das Betätigungselement zumindest teilweise durch das Verschieben in das von der Einstichöffnung abgewandte Ende des Gehäuses, im Folgenden als "distales Ende" des Gehäuses bezeichnet, eingeführt. Dabei ist das Betätigungselement weiterhin bevorzugt so angeordnet ist, dass das Verschieben entlang eines vorzugsweise linearen Betätigungsweges erfolgt. Wiederum bevorzugt verläuft dieser vorzugsweise lineare Betätigungsweg parallel zur Einstichbewegung der Lanzette, d.h. bevorzugt parallel zur Hauptachse (A) des Gehäuses. Im Rahmen einer erfindungsgemäß bevorzugten Ausführungsform stellt das Betätigungselement einen aus dem hinteren, von der Austrittöffnung für die Lanzettenspitze abgewandten, d.h. distalen Ende des Gehäuses herausragenden Bedienknopf dar.

Das Betätigungselement, vorzugsweise der wie vorstehend dargestellt Bedienknopf, weist erfindungsgemäß einen Ausgangszustand und einen betätigten Zustand auf und kann durch Verschieben vom Ausgangszustand in den betätigten Zustand überführt werden. Im Rahmen einer erfindungsgemäß besonders bevorzugten Ausführungsform kann der Bedienknopf in Richtung des proximalen Endes des Gehäuses in das Gehäuse eingeführt bzw. eingedrückt werden.

Erfindungsgemäß bevorzugt ist die kombinierte Spann- und Auslösevorrichtung mit dem Lanzettenantrieb derartig gekoppelt, dass das elastische Antriebselement des Lanzettenantriebs durch stetiges, in eine Richtung gerichtetes Verschieben des Betätigungselementes zunächst aus einem ungespannten in einen gespannten Zustand überführt wird und anschließend, vorzugsweise automatisch, ausgelöst wird. Dabei bezeichnet der Begriff "stetig" im Rahmen der vorliegenden Erfindung eine solche Bewegung, die kontinuierlich, d.h. ohne zwischenzeitlichen Stillstand des bewegten bzw. verschobenen Elementes vorgenommen wird, wobei es möglich ist, dass die Geschwindigkeit der Bewegung variieren kann. Unter dem Begriff "in eine Richtung gerichtet" ist im Rahmen der vorliegenden Erfindung eine Bewegung des Betätigungselementes zu verstehen, die entlang des Betätigungsweges geführt wird, ohne die Bewegungsrichtung zu ändern, wobei der Betätigungsweg vorzugsweise linear verläuft und besonders bevorzugt parallel zur Einstichrichtung bzw. zur Hauptachse (A) des Gehäuses verläuft.

Im Rahmen einer erfindungsgemäß insbesondere bevorzugten Ausführungsform weist das Entnahmegerät einen Bedienknopf auf, der aus dem distalen Ende des Gehäuses herausragt und durch Eindrücken in Richtung des proximalen Endes des Gehäuses ganz oder zumindest teilweise in das Gehäuse verschoben werden kann.

Durch die dargestellte Betätigung des Betätigungselementes, bevorzugt durch Eindrücken des Bedienknopfes am distalen Ende des Gehäuses in Richtung des proximalen Endes des Gehäuses kann erfindungsgemäß das elastische Antriebselement des Lanzettenantriebs zunächst aus einem ungespannten in einen gespannten Zustand überführt und anschließend, vorzugsweise unmittelbar anschließend, automatisch ausgelöst werden. Die Auslösung des Stechvorganges erfolgt erfindungsgemäß bei Erreichen eines vorbestimmten Punktes, den das Betätigungselement im Verlauf der Verschiebung aus dem Ausgangszustand in den betätigten Zustand erreicht und der vorzugsweise durch Konstruktion der Arretiervorrichtung vorbestimmt ist. Dadurch kann erreicht werden, dass die Auslösung des Stechvorganges unabhängig von der zur Spannung der Feder aufzubringenden Kraft erfolgt.

Unter dem Begriff "automatisch" ist dabei zu verstehen, dass zum Auslösen des Antriebselementes, d.h. zum Auslösen der Einstichbewegung von Lanzette bzw. Lanzettenhalter, keine weiteren Operationen notwendig sind als das dargestellte stetige, in eine Richtung gerichtete Verschieben bzw. Eindrücken des Betätigungselementes.

Dabei kann in Abhängigkeit der speziellen Ausgestaltung der kombinierten Spann- und Auslöseeinrichtung frei gewählt werden, welcher Anteil der Verschiebung des Betätigungselementes entlang des Betätigungsweges zunächst zum Spannen des Lanzettenantriebs genutzt wird bzw. bei Erreichen welchen Punktes auf dem Betätigungsweg das Auslösen des Lanzettenantriebes erfolgt. So ist es beispielsweise denkbar, dass das Betätigungselement zunächst fast über den gesamten Betätigungsweg verschoben werden muss und das Auslösen erst bei oder vorzugsweise kurz vor Erreichen der maximal möglichen Verschiebung erreicht wird. Alternativ ist es beispielsweise auch möglich, dass bei Erreichen der Hälfte des maximalen Betätigungsweges des Bedienelementes bereits die Auslösung des Lanzettenantriebs erfolgt, wodurch allerdings eine kürzere Verschiebung des Betätigungselementes zum Spannen des Lanzettenantriebes und damit ein höherer Kraftaufwand verbunden wäre. Vorteilhaft ist dabei, dass dabei während der Betätigung des Bedienelementes nicht feststellbar ist, wann, d.h. zu welchem Zeitpunkt während des Verschiebens bzw. Eindrückens des Betätigungselementes, ein Auslösen des Einstichvorganges erfolgt Gewünschtenfalls kann das erfindungsgemäße System jedoch auch geeignete Mittel zur Anzeige des bevorstehenden Auslösens der Einstichbewegung umfassen. Diese geeigneten Mittel könnten beispielsweise sichtbare oder ertastbare Markierungen auf dem Gehäuse des Entnahmesystems oder auf der Oberfläche des Bedienelementes sein. Besonders bevorzugt wären die genannten Mittel zur Anzeige des bevorstehenden Auslösens der Einstichbewegung Teil des Bedienelementes, besonders bevorzugt eine sicht- oder ertastbare Markierung auf der Oberfläche des Bedienelementes, beispielsweise ein farblich oder durch die Oberflächenbeschaffenheit vom Rest des Bedienelementes unterscheidbarer Bereich, vorzugsweise am distalen Ende des Betätigungselementes.

Die kombinierte Spann- und Auslösevorrichtung weist erfindungsgemäß eine Arretiervorrichtung auf. Grundsätzlich sind verschiedene technische Ausgestaltungen der erfindungsgemäß vorgesehenen Arretiervorrichtung denkbar, bei der die mechanische Kopplung zwischen Betätigungselement und Lanzettenantrieb so ausgestaltet ist, dass der Lanzettenantrieb bei Verschieben des Betätigungselementes entlang des Betätigungsweges zunächst gespannt und dann ausgelöst wird, wobei die Auslösung des Lanzettenantriebs bei Erreichen eines bestimmten Punktes entlang des Betätigungsweges, wie vorstehend erläutert, freigegeben wird.

Beispielsweise kann durch ein Eingreifen eines mit dem Betätigungselement fest verbundenen Haltelementes in den Lanzettenantrieb bewerkstelligt werden, dass dieses zumindest abschnittsweise bei einer Verschiebung des Betätigungselementes entlang des Betätigungsweges in den Antriebsrotor des Drehschiebergetriebes eingreift und dadurch dessen Drehbarkeit zumindest einschränkt, vorzugsweise verhindert.

Im Rahmen einer besonders bevorzugten Ausführungsform umfasst das erfindungsgemäße System, konkret die Entnahmevorrichtung, eine Arretiervorrichtung, die als Bahnsteuerung ausgestaltet ist, welche als Glieder einen Steuerbahnteil und einen Steuernocken umfasst, wobei der Steuernocken während mindestens eines Teils der Verschiebung des Betätigungselementes entlang des Betätigungsweges eine Relativbewegung bezüglich des Steuerbahnteils macht, bei der er die Steuerbahn des Steuerbahnteils zumindest teilweise, bevorzugt vollständig abfährt, wodurch bevorzugt mindestens ein Teil der Bewegung des Lanzettenantriebs gesteuert wird.

Im Rahmen einer bevorzugten Ausführungsform ist der Steuernocken ein Bestandteil des Betätigungselementes oder fest mit dem Betätigungselement verbunden. Weiterhin bevorzugt ist der Steuerbahnteil ein Bestandteil des Lanzettenantriebes oder fest mit einem Bestandteil des Lanzettenantriebes verbunden. Im Rahmen einer erfindungsgemäß insbesondere bevorzugten Ausführungsform ist der Steuerbahnteil der Bahnsteuerung ganz oder zumindest teilweise, vorzugsweise jedoch vollständig, Bestandteil des Antriebsrotors bzw. auf dem Antriebsrotor eines wie vorstehend beschriebenen Lanzettenantriebes angebracht. Dabei hat es sich als vorteilhaft erwiesen, wenn die Steuerbahn auf der Außenseite des Antriebsrotors angebracht ist, wobei sie prinzipell auch auf andere Weise mit diesem verbunden sein kann, beispielsweise auf der nach innen gewandten Oberfläche eines gegebenenfalls hohlen bzw. hülsenförmig ausgestalteten Antriebsrotors.

Bevorzugt führt der Steuernocken während der gesamten Verschiebung des Betätigungselementes aus der Ausgangsposition in die betätigte Position eine Relativbewegung bezüglich des Steuerbahnteils durch. Dabei fährt der Steuernocken die Steuerbahn zumindest teilweise, bevorzugt jedoch vollständig ab. Die Steuerbahn kann dabei beispielsweise als Ausnehmung in der Oberfläche des jeweiligen Bestandteils des Lanzettenantriebs ausgestaltet sein. Es ist in bevorzugter Weise jedoch auch möglich, die Steuerbahn in Form einer über die Oberfläche des jeweiligen Elementes, bevorzugt über die Oberfläche des Antriebsrotors hinausragende Schiene oder auch Form zweier, im Wesentlichen parallel zueinander geführter Schienen auszugestalten. An einer derartigen Schiene bzw. in einer durch zwei Schienen gebildeten Bahn kann der Steuernocken dann entlang gleiten. Im Rahmen einer bevorzugten Ausführungsform umfasst die Steuerbahn zumindest einen linearen Abschnitt der vorzugsweise parallel zur Drehachse des Lanzettenantriebs bzw. bevorzugt parallel zur Drehachse des Antriebsrotors verläuft, sofern sie als Bestandteil des Antriebsrotors ausgestaltet ist.

In einer weiterhin bevorzugten Ausführungsform umfasst der Steuerbahnteil einen ersten, vorzugsweise linearen Abschnitt und einen zweiten, vorzugsweise linearen Abschnitt, wobei der erste Abschnitt des Steuerbahnteils im Wesentlichen parallel zur Drehachse des erfindungsgemäß bevorzugten Drehschiebergetriebes, d.h. bevorzugt parallel zur Drehachse des Antriebsrotors verläuft und wobei der zweite Abschnitt des Steuerbahnteils im Wesentlichen senkrecht zur Drehachse des Drehschiebergetriebes, d.h. bevorzugt senkrecht zur Drehachse des Antriebsrotors verläuft. Unter den genannten Begriffen "im Wesentlichen parallel" bzw. "im Wesentlichen senkrecht" sind dabei wie im Rahmen der gesamten Offenbarung geringe Abweichungen von bis zu etwa 10°, bevorzugt von bis zu etwa 5° und besonders bevorzugt bis zu etwa 2° (jeweils in beide Richtungen) von der idealen parallelen bzw. senkrechten Ausrichtung zu verstehen.

Der erste Abschnitt des Steuerbahnteils verläuft bevorzugt im Wesentlichen parallel, bevorzugt parallel zur Drehachse des Drehschiebergetriebes bzw. Antriebsrotors sofern auf diesem angebracht, d.h. vorzugsweise parallel zur Hauptachse (A) des Gehäuses. Der zweite Abschnitt des Steuerbahnteils verläuft bevorzugt im Wesentlichen senkrecht, bevorzugt senkrecht zur Drehachse des Drehschiebergetriebes bzw. Antriebsrotors, sofern auf diesem angebracht. Vorzugsweise sind die beiden Abschnitte, d.h. der im Wesentlichen parallel und der im Wesentlichen senkrecht zur Drehachse des Drehschiebergetriebes bzw. Antriebsrotors verlaufende Abschnitt unmittelbar miteinander verknüpft, so dass sie vom Steuernocken kontinuierlich abgefahren werden können, wenn das Betätigungselement entlang des Betätigungsweges durch stetiges, in eine Richtung gerichtetes Verschieben von einem Ausgangszustand in einen betätigten Zustand überführt wird. Die gebildete Steuerbahn weist in dieser Ausgestaltung einen im Wesentlichen rechtwinkligen Verlauf auf. Dabei wird insbesondere bevorzugt zuerst der im Wesentlichen parallel zur Geräteachse, bevorzugt parallel zur Drehachse des Drehschiebergetriebes bzw. Antriebsrotors verlaufende Teil des Steuerbahnteils vom Steuernocken abgefahren. Dadurch kann gewährleistet werden, dass das im Verlauf des Verschiebens des Betätigungselementes bewirkte Spannen des Antriebselementes unmittelbar in ein Auslösen des so gespannten Lanzettenantriebes übergeht und somit die Einstichbewegung des Lanzettenhalters bzw. der darin gehaltenen Lanzette ohne weitere Bedienschritte lediglich durch das Erreichen des durch das Ende des ersten, im Wesentlichen parallel zur Drehachse des Antriebsrotors verlaufenden Abschnitts der Steuerbahn, bzw. durch den Anfang des zweiten, im Wesentlichen senkrecht zur Drehachse des Antriebsrotors ausgerichteten Steuerbahnteils ausgelöst wird. Dies wird im Rahmen dieser Ausführungsform dadurch gewährleistet, dass der Lanzettenantrieb, konkret der Antriebsrotor, sofern die Steuerbahn auf diesem angebracht ist, während des Spannens zunächst in seiner Rotation gehemmt ist, da der Steuernocken in den im Wesentlichen parallel zur Drehachse verlaufenden Abschnitt des Steuerbahnteils eingreift. Mit Erreichen des Endes des im Wesentlichen parallel verlaufenden Abschnitts bzw. mit Erreichen des zweiten, im Wesentlichen senkrecht zur Rotationsachse verlaufenden Abschnitts wird dann die Rotation des Antriebsrotors freigegeben. Die erfindungsgemäß vorgesehene Auslösung der Einstichbewegung bei Erreichen eines vorbestimmten Punktes entlang des Verschiebungsweges des Betätigungselementes wird im Rahmen dieser Ausführungsform durch das Ende des ersten, parallel zur Rotationsachse des Antriebsrotors verlaufenden Abschnittes des Antriebsrotors bestimmt, bzw. durch den Punkt, an dem der erste Abschnitt der Steuerbahn in den zweiten, senkrecht zur Rotationsachse des Antriebsrotors verlaufenden Abschnitt der Steuerbahn übergeht. Unter dem Begriff "senkrecht zur Drehachse" bzw. "senkrecht zur Rotationsachse A" verlaufend ist dabei selbstverständlich auch ein entlang einer kreisförmigen Bahn um den Lanzettenantrieb bzw. ein kreisförmig entlang des Umfanges des Antriebsrotor verlaufender Abschnitt der Steuerbahn zu verstehen.

In einer weiteren vorteilhaften Ausführungsform kann die Steuerbahn neben dem vorstehend genannten ersten und zweiten Abschnitt auch weitere Abschnitte umfassen, die nicht im Wesentlichen parallel bzw. senkrecht zur Drehachse des Antriebsrotors ausgerichtet sind. So kann es beispielsweise vorteilhaft sein, Bahnabschnitte vorzusehen, die weder parallel noch senkrecht zur Drehachse des Antriebsrotors ausgerichtet sind, sondern mit dieser einen Winkel von bis zu etwa 60°, bevorzugt bis zu etwa 40° und besonders bevorzugt bis zu etwa 20° einschließen. Derartige Steuerbahnabschnitte können beispielsweise zwischen den vorstehend genannten ersten und zweiten abschnitten der Steuerbahn vorgesehen werden, wodurch bei der Betätigung des Betätigungselementes die bevorstehende Freigabe des Antriebsrotors, d.h. der bevorstehende Einstich, signalisiert werden kann.

Wie vorstehend ausgeführt wird das Betätigungselement im Rahmen einer bevorzugten Ausführungsform entlang des Betätigungsweges geführt, ohne die Bewegungsrichtung zu ändern, wobei der Betätigungsweg vorzugsweise linear verläuft und besonders bevorzugt parallel zur Einstichrichtung bzw. zur Hauptachse (A) des Gehäuses verläuft und besonders bevorzugt in Richtung des proximalen Endes des Gehäuses geführt wird. Nach erfolgter Einstichbewegung des Lanzettenhalters bzw. der damit lösbar fest verbundenen Lanzette wird dann das Betätigungselement üblicherweise in seine Ausgangsposition, vorzugsweise in Richtung des distalen Endes des Entnahmegeräts zurückverschoben, beispielsweise durch Einwirkung einer geeigneten Rückstellfeder, wie es dem Fachmann geläufig ist. Dabei bewegt sich der Lanzettenantrieb bzw., falls vorgesehen, der Antriebsrotor, wieder in seine Ausgangsposition zurück, so dass der nächste Einstichvorgang durchgeführt werden kann.

Im Rahmen einer weiteren bevorzugten Ausführungsform umfasst das erfindungsgemäße System einen Lanzettenantrieb mit Drehschiebergetriebe, das ein drehbares eingangsseitiges Getriebeglied aufweist, durch welches ein auf der Eingangsseite des Drehschiebergetriebes eingeleitetes Drehmoment in eine Längsverschiebung in Richtung des vorbestimmten Einstichweges umgewandelt wird. Vorteilhaft ist der Lanzettenantrieb mechanisch so mit dem Betätigungselement verknüpft, dass auf der Eingangsseite des Drehschiebergetriebes die Verschiebung des Betätigungselementes längs des Betätigungsweges in eine Drehbewegung des drehbaren Getriebegliedes umgesetzt wird, um das drehbare Getriebeglied gegen die Kraft des elastischen Antriebselementes zu spannen. Dabei kann die Rotationsachse des drehbaren Getriebegliedes sowohl senkrecht als auch parallel zur Einstichrichtung bzw. zur Hauptachse (A) des Gehäuses ausgerichtet sein. Bevorzugt ist die Rotationsachse des drehbaren Getriebegliedes des Drehschiebergetriebes parallel zur Einstichrichtung bzw. zur Hauptachse (A) des Gehäuses ausgerichtet. Dies kann beispielsweise dadurch bewerkstelligt werden, dass die Eingangsseite des Drehschiebergetriebes mittels einer an dem drehbaren Getriebeglied vorgesehenen Wendel und mittels eines mit dem Betätigungselement verbundenen, längs des Betätigungsweges beweglichen und auf einer Gleitfläche der Wendel mittels einer Kontaktfläche gleitbaren Spannnockens ausgebildet ist. Eine derartige Vorrichtung ist beispielsweise aus der EP 1 034 740 A1 bekannt, auf die hiermit diesbezüglich Bezug genommen wird. Im Rahmen einer bevorzugten Ausführungsform ist der Spannocken fest mit dem erfindungsgemäß vorgesehenen Betätigungselement verbunden.

Das erfindungsgemäße System umfasst, wie bereits dargelegt, ein Entnahmegerät und eine Mehrzahl von auf das Entnahmegerät abgestimmten Lanzetten. Dabei ist unter einer Mehrzahl im Rahmen der vorliegenden Erfindung in der Regel eine Menge von 2 bis etwa 50, bevorzugt 3 bis etwa 25, besonders bevorzugt 4 bis 10, ganz besonders bevorzugt 5 bis 8, noch mehr bevorzugt 6 oder 7 und am meisten bevorzugt 6 Lanzetten zu versehen. Die Lanzetten werden erfindungsgemäß auswechselbar durch den Lanzettenhalter gehaltert, so dass sie nach ein- oder gewünschtenfalls mehrmaligem Gebrauch vom Lanzettenhalter gelöst und durch eine andere, vorzugsweise ungebrauchte Lanzette ausgetauscht werden. Im Hinblick nicht zuletzt der besseren Handhabbarkeit hat es sich als vorteilhaft erwiesen, mehrere Lanzetten in magazinierter Form bereitzustellen und ein derartiges Lanzettenmagazin im Rahmen des erfindungsgemäßen Systems vorzusehen.

Das erfindungsgemäße System umfasst daher ein Magazin mit einer Mehrzahl von Lanzetten, die nacheinander an den Lanzettenhalter angekoppelt werden können. Derartige Magazine, oft auch als "Stecheinheiten" bezeichnet, sind dem Fachmann bekannt und beispielsweise in der WO 02/36010 A1 offenbart, auf die diesbezüglich ausdrücklich Bezug genommen wird. Die erfindungsgemäß bevorzugt einsetzbaren Magazine bzw. Stecheinheiten umfassen mehrere Lanzetten, die sich üblicherweise jeweils einzeln in separierten Kammern befinden, die von einem Magazingehäuse umgeben sind. Die Lanzetten befinden sich in Ruheposition, d.h. vor der Einstichbewegung sowie vorzugsweise nach der Einstichbewegung bevorzugt innerhalb des Magazingehäuses. Auf diese Weise können ungewollte Verletzungen insbesondere mit einer bereits benutzten Lanzette vermieden werden.

Das Magazin ist vorteilhaft so ausgestaltet, dass es in das Gehäuse des vorgesehenen Entnahmegerätes eingebracht werden kann. Weiterhin vorteilhaft ist das Lanzettenmagazin so ausgestaltet, dass es an dem Lanzettenantrieb angebracht werden kann. Hierzu kann das erfindungsgemäß einsetzbare Magazin beispielsweise die Form einer Kappe aufweisen, die auf den Lanzettenantrieb aufgesteckt wird. Erfindungsgemäß bevorzugt einzusetzende Magazine weisen in der Regel mehrere Kammern auf, in denen sich jeweils eine Lanzette befindet, die nacheinander relativ zum Lanzettenantrieb bzw. Lanzettenhalter positioniert werden, so dass die Lanzette an den als Stößel ausgestalteten Lanzettenhalter angekoppelt werden kann. Die genannten Kammern können dazu beispielsweise reihenförmig nebeneinander angeordnet sein. Vorteilhaft setzt man im Rahmen des erfindungsgemäßen Systems jedoch ein rotationssymmetrisches Magazin ein. Derartige, auch als Trommelmagazine bezeichnete Magazine weisen parallel zu deren Rotationsachse angeordnete Kammern auf und werden vorteilhaft so in das erfindungsgemäße System eingebracht, dass ihre Rotationsachse parallel, besonders bevorzugt koaxial zur Hauptachse (A) des Systemgehäuses verläuft. Ähnlich einer Revolvertrommel kann ein solches Magazin automatisch oder manuell repetierbar an der Antriebseinheit angebracht sein.

Im Rahmen einer erfindungsgemäß bevorzugten Ausführungsform umfasst das Magazin zumindest einen Teil der Lanzettenführung, besonders bevorzugt die gesamte Lanzettenführung, wie beispielsweise in der vorstehend genannten WO 02/36010 A1 offenbart, auf die hiermit diesbezüglich Bezug genommen wird.

Konstruktionsbedingt führt das vorstehend beschriebene Prinzip der Magazinierung von Lanzetten oft dazu, dass der im Verlauf des Einstichvorgangs zurückzulegende Weg der mit dem hanzettenantrieb gekoppelten Lanzette sich im Vergleich zu dem einer nicht magazinierten Lanzette merklich verlängert. Dies führt in der Regel auch zu größeren, d.h. ungünstigeren Abmessungen derartiger Entnahmesysteme im Vergleich zu Systemen ohne magazinierte Lanzetten.

Im Rahmen einer weiterhin bevorzugten Ausführungsform sind die erfindungsgemäß vorgesehene Lanzette oder die vorgesehenen Lanzetten mit je einem Sterilschutz versehen, der die Sterilität von noch unbenutzten Lanzetten, zumindest der jeweiligen Lanzettenspitzen bis zum Einstich gewährleistet. Geeignete Sterilschutzkörper sind beispielsweise solche, die vor der Benutzung von der Lanzette abgezogen werden. Besonders bevorzugt sind im Rahmen der vorliegenden Erfindung solche Sterilschutzkörper, die in der der Regel aus einem elastomeren Material bestehen und beim Einstich durch die zu schützende Lanzettenspitze durchstochen oder abgestreift, bevorzugt jedoch durchstochen werden, wie sie aus der EP 1263 320 bekannt sind. In derartigen Systemen sind in der Regel höhere Antriebskräfte notwendig, da ein zusätzlicher Krafteintrag zum Abstreifen oder Durchstechen des üblicherweise elastischen Sterilschutzes notwendig ist.

Die erfindungsgemäßen Systeme zur Entnahme von Körperflüssigkeiten ermöglichen, insbesondere durch die erfindungsgemäß vorgesehene Arretiervorrichtung, eine deutlich kompaktere Bauweise auch bei Verwendung magazinierter und insbesondere zusätzlich mit individuellem Sterilschutz versehenen Lanzetten.

Ein weiterer Vorteil der erfindungsgemäßen Systeme zur Entnahme von Körperflüssigkeiten besteht darin, dass der Lanzettenantrieb bzw. das Antriebselement nicht wie bei vergleichbaren Geräten des Standes der Technik bei der Benutzung zunächst durch Spannen in einen gespannten Zustand versetzt wird und dauerhaft bzw. für einen frei bestimmbaren Zeitraum in diesem gespannten Zustand verbleibt bis er in einem separaten Bedienschritt ausgelöst wird. Eine derartige Verrastung bzw. Arretierung des Lanzettenantriebes im gespannten Zustand geht üblicherweise damit einher, dass zum sicheren Verrasten der Arretierung sowie zum Lösen der Arretierung, d.h. zum Auslösen des Stehvorganges ein zusätzlicher Energieeintrag durch den Benutzer aufzubringen ist. Beim bevorzugten 360°-Antrieb wird dieser zusätzliche Energieeintrag beispielsweise durch einen zusätzlich erforderlichen Drehwinkel erzeugt. Durch die erfindungsgemäße Kopplung des Spann- und Auslösevorganges kann daher eine leichtere Bedienbarkeit erreicht werden, da kein derartiger zusätzlicher Kraftaufwand durch den Benutzer zu leisten ist. Dementsprechend erlaubt die vorliegende Erfindung zur Erzielung der gleichen Antriebskräfte bzw. -geschwindigkeiten auch den Einsatz von leistungsschwächeren Antriebselementen. Insbesondere beim Einsatz von Spiralfedern als Antriebselemente können dann solche mit geringerer Kraftkonstante eingesetzt werden. Dies bringt deutliche Vorteile im Hinblick auf einen geringeren, durch den Benutzer zu leistenden Kraftaufwand, eine geringere mechanische Gesamtbelastung des Systems sowie im Hinblick auf verringerte Herstellkosten mit sich.

Darüber hinaus hat sich gezeigt, dass insbesondere bei Einsatz von Spiralfedern mit geringerer Kraftkonstante unerwünschte Nebengeräusche reduziert werden können. Derartige Nebengeräusche treten üblicherweise bei der Entspannung gespannter Spiralfedern auf und entstehen durch das Aneinaderschlagen der Federwindungen während der Entspannungsbewegung, insbesondere bei Spiralfedern, die durch axiale Torsion um die Längsachse der Spiralfeder gespannt bzw. entspannt werden.

Weiterhin führt auch das Fehlen einer weiteren Verrastung des Lanzettenantriebes im gespannten Zustand in Abhängigkeit von der gewählten mechanischen Ausgestaltung üblicherweise zu einer deutlich wahrnehmbaren Geräuschreduktion während der Bedienung des erfindungsgemäßen Lanzettensystems. Es hat sich gezeigt, dass ein wie beschrieben verringerten Bediengeräusche des erfindungsgemäßen Lanzettensystems den Bedienkomfort, insbesondere im Hinblick auf eine diskrete Benutzung deutlich erhöhen und somit zu einer deutlich verbesserten Akzeptanz beim Benutzer führen.

Ein weiterer Aspekt der vorliegenden Erfindung betrifft auch ein wie vorstehend beschriebenes Entnahmegerät (1), dass sich zur Bereitstellung des erfindungsgemäßen Systems zur Entnahme von Körperflüssigkeiten für Diagnosezwecke eignet. Ein derartiges Entnahmegerät umfasst vorzugsweise
- ein Gehäuse (10) mit einer Austrittsöffnung für die Lanzettenspitze einer auf das Entnahmegerät abgestimmten Lanzette,
- einen in dem Gehäuse (10) entlang eines vorbestimmten Einstichweges beweglichen Lanzettenhalter (20) zur auswechselbaren Halterung der Lanzette (91),
- eine Lanzettenführung (92) zur Führung des Lanzettenhalters (20) auf dem vorbestimmten Einstichweg nach Auslösen der Einstichbewegung,
- einen Lanzettenantrieb (30) mit einem elastischen Antriebselement (31), der sich durch Spannen aus einem ungespannten in einen gespannten Zustand überführen lässt und durch den nach dem Auslösen die Entspannungsbewegung des gespannten elastischen Antriebselementes (31) in eine Einstichbewegung umgesetzt wird, bei der die vom Lanzettenhalter (20) gehaltene Lanzette (91) entlang des vorbestimmten Einstichweges in Einstichrichtung bewegt wird, bis die Lanzettenspitze zumindest teilweise aus der Austrittsöffnung austritt, und durch den der Lanzettenhalter (20) in eine Position zurückgeführt wird, bei der die Spitze der Lanzette (91) sich in dem Gehäuse (10) befindet, und
- eine kombinierte Spann- und Auslöseeinrichtung mit einem von der Außenseite des Gehäuses (10) zugänglichen Betätigungselement (40), das einen Ausgangszustand und einen betätigten Zustand aufweist, wobei die kombinierte Spann- und Auslöseeinrichtung eine Arretiervorrichtung (70) aufweist, die mit dem Betätigungselement (40) und dem Lanzettenantrieb (30) derart mechanisch gekoppelt ist, dass der Lanzettenantrieb (30) bei Verschieben des Betätigungselementes (40) entlang des Betätigungsweges zunächst gespannt und dann ausgelöst wird, wobei die Auslösung des Lanzettenantriebs (30) bei Erreichen eines bestimmten Punktes entlang des Betätigungsweges freigegeben wird.

Die Erfindung wird nachfolgend anhand von in den Figuren dargestellten Ausführungsbeispielen näher erläutert. Dabei können die in den einzelnen Figuren dargestellten Besonderheiten unter Schaffung bevorzugter Ausführungsformen der vorliegenden Erfindung frei miteinander kombiniert werden. Es zeigen:
- Fig. 1a: einen Querschnitt eines erfindungsgemäßen Systems mit aufgesetztem Lanzettenmagazin;
- Fig. 1b: eine Ausschnittsvergrößerung des Lanzettenmagazins des Querschnitts aus Fig. 1a
- Fig. 2: eine perspektivisch dargestellter Querschnitt des Systems aus Fig. 1a;
- Fig. 3: eine perspektivische Darstellung eines Entnahmegeräts des erfindungsgemäßen Systems ohne Gehäuse;
- Fig. 3a: gesonderte Darstellung der Arretiervorrichtung
- Fig. 4: eine Seitenansicht des Entnahmesystems aus Fig. 3 vor dem Verschieben des Betätigungselementes, d.h. im Ausgangszustand;
- Fig. 5: eine Seitenansicht des Entnahmegeräts aus Fig. 3, wobei sich das Betätigungselement im betätigten Zustand befindet;
- Fig. 6: eine Seitenansicht des Entnahmegeräts aus Fig. 3, mit bereits rotiertem Antriebsrotor und in Einstichrichtung verschobenem Lanzettenhalter;
- Fig. 7: eine Seitenansicht der rückwärtigen Seite des in Fig. 6 dargestellten Entnahmegeräts;
- Fig. 8: eine Detailansicht des ausgangsseitigen Getriebegliedes mit Lanzettenhalter des in den Fig. 3 bis 7 dargestellten Entnahmegeräts;
- Fig. 9: vereinfachte Darstellung der Detailansicht des ausgangsseitigen Getriebegliedes mit Lanzettenhalter des in den Fig. 3 bis 7 dargestellten Entnahmegeräts ohne Antriebsrotor;
- Fig. 10: eine perspektivische Darstellung des in den Fig. 8 und 9 dargestellten Lanzettenhalters
- Fig. 11: eine perspektivische Darstellung des in Fig. 1a dargestellten Entnahmegerätes mit aufgesetztem Lanzettenmagazin ohne Gehäuse.

### Liste der Bezugszeichen:

- 1: Entnahmegerät
- 2: proximales Ende des Entnahmesystems
- 3: distales Ende des Entnahmesystems
- 10: Gehäuse
- 11: proximales Ende des Gehäuses
- 12: distales Ende des Gehäuses
- 20: Lanzettenhalter/ Stößel
- 21: Kopplungsgeometrie (Haltevorrichtung) des Lanzettenhalters
- 30: Lanzettenantrieb
- 31: elastisches Antriebselement
- 40: Betätigungselement
- 50: Antriebsrotor
- 60: Kurvensteuerung
- 61: Stechkurve/Stechprofil
- 62: Steuerzapfen
- 63: Vortriebshülse
- 70: Arretiervorrichtung
- 71: Steuernocken
- 72: erster Abschnitt des Steuerbahnteils
- 73: zweiter Abschnitt des Steuerbahnteils
- 80: wendelförmiger Spannbolzen
- 81: Spannnocken
- 82: Spannhülse
- 90: Lanzettenmagazin
- 91: Lanzette
- 92: Lanzettenführung
- 93: Haltebereich der Lanzette
- 94: Sterilschutz

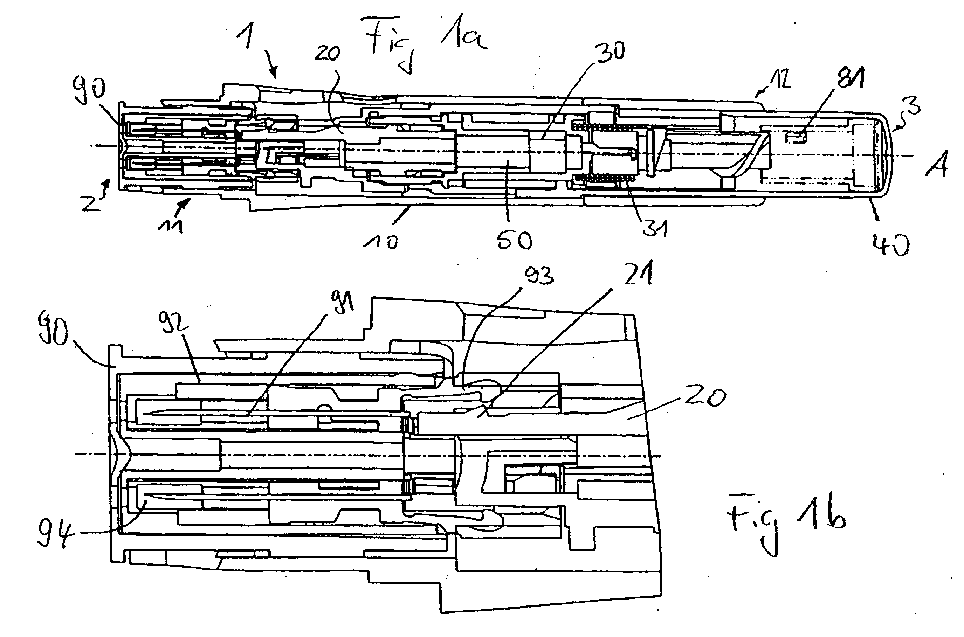

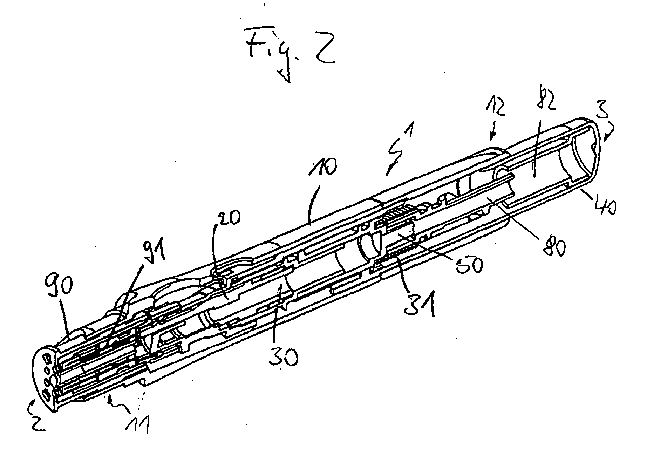

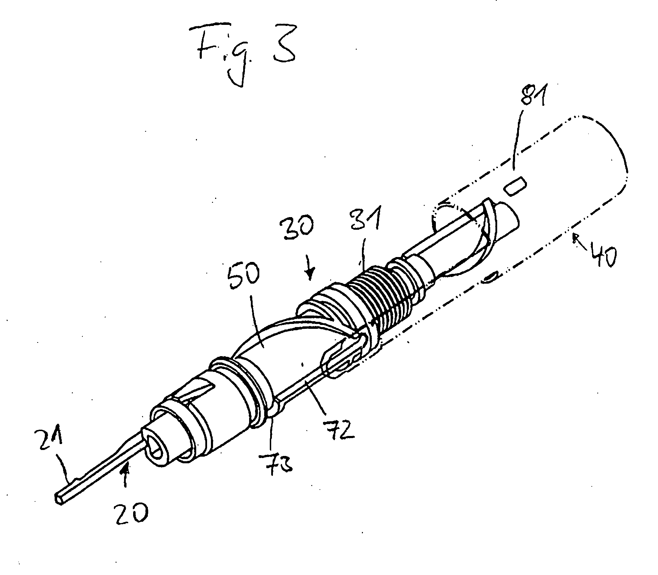

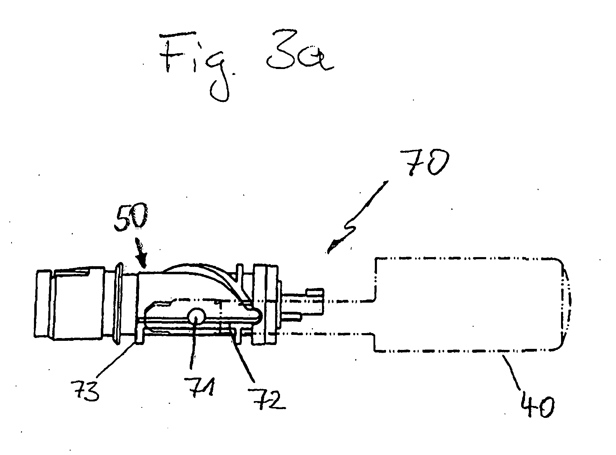

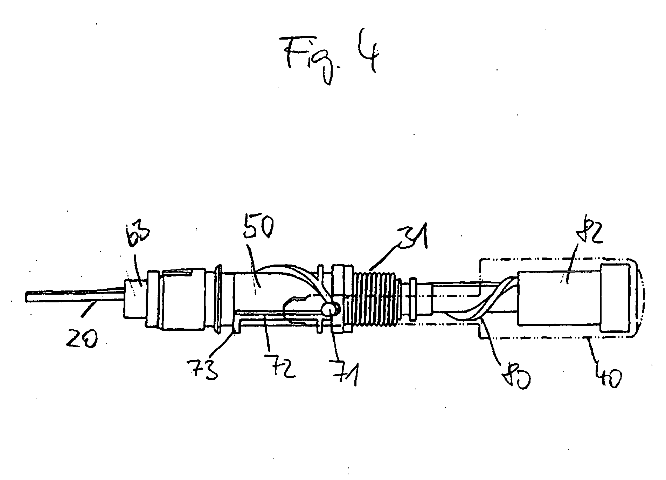

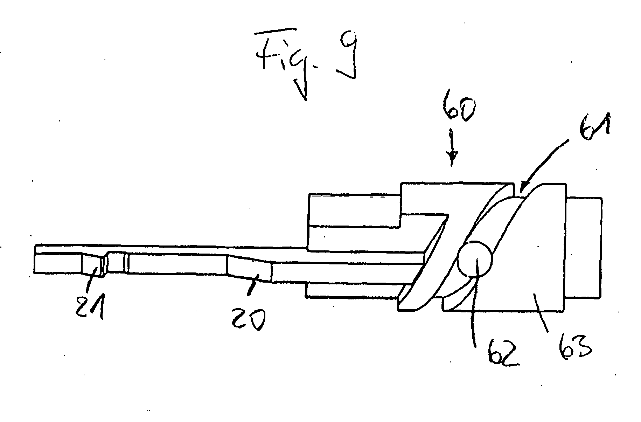

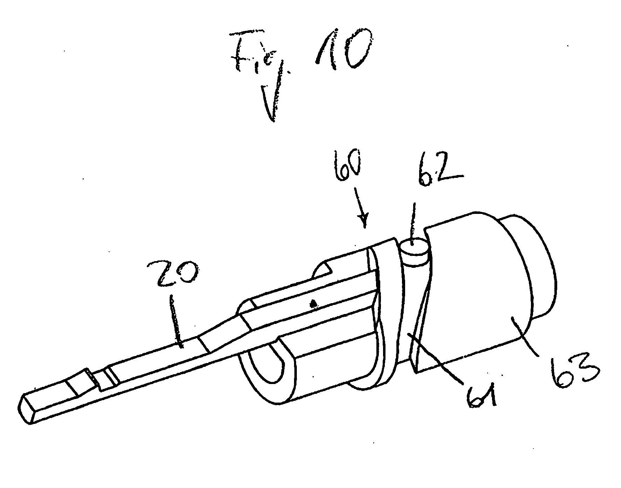

Die Figuren 1 und 2 stellen Querschnitte entlang der Hauptdrehachse bzw. Hauptgeräteachse (A) eines erfindungsgemäßen Systems, das in der dargestellten Form ein Entnahmegerät (1) und ein Lanzettenmagazin (90) umfasst. Die Figuren 3 bis 11 zeigen Details des Entnahmegerätes, teilweise in seitlicher, teilweise in perspektivischer Ansicht. Das Lanzettenmagazin (90) bildet das proximale Ende (2) des Systems und ist in das proximale Ende (11) des Gehäuses (10) eingefügt. Das Betätigungselement (40) bildet das distale Ende (3) des Systems und ragt aus dem distalen Ende (12) des Gehäuses (10) heraus. Das System weist einen Lanzettenantrieb (30) in Form eines Drehschiebergetriebes auf, der mit einer als Torsionsfeder ausgestalteten Spannfeder als elastisches Antriebselement (31) über einen Antriebsrotor (50) verbunden ist. Der Lanzettenantrieb ist ausgangsseitig mit einem als Stößel ausgebildeten Lanzettenhalter (20) verbunden, der an seinem proximalen Ende eine Haltevorrichtung (21) zur lösbaren Halterung einer Lanzette (91) durch formschlüssige Kopplung mit deren Haltebereich (93) aufweist und über eine Vortriebshülse (63, siehe Figuren 4 bis 10) mit dem eingangsseitigen Getriebeglied (50) verbunden ist. Das in dieser Ausführungsform vorgesehene Lanzettenmagazin (90) umfasst eine Lanzettenführung (92), durch die der Lanzettenhalter bzw. die damit lösbar verbundene Lanzette (91) auf einem vorbestimmten Einstichweg parallel zur Drehachse (A) des Systems geführt werden. Die im Lanzettenmagazin bevorrateten Lanzetten weisen je einen Sterilschutz (94) auf, der zumindest die jeweilige Lanzettenspitze dicht umgibt. Die Details sind in der in Fig. 1b dargestellten Ausschnittsvergrößerung verdeutlicht. In dieser Ausführungsform ist die Lanzettenführung (92) als Bestandteil eines Lanzettenmagazins (90) ausgestaltet. In Fällen, in denen kein derartiges Lanzettenmagazin vorgesehen ist, d.h. bei erfindungsgemäßen Systemen mit Einzellanzetten, kann die Lanzettenführung selbstverständlich auch anders, beispielsweise als Bestandteil des Gehäuses, sein, d.h. beispielsweise auf der Innenseite des Gehäuses angebracht sein.

Der Antriebsrotor (50) des Drehschiebergetriebes (Ausgangsseite) ist in einer festen Axialposition bezüglich der Geräteachse (A) gelagert und über die Antriebsfeder (31) mit einem als wendelförmigem Spannbolzen (80) ausgestalteten eingangsseitigen Getriebeglied verknüpft. Der Spannbolzen (80) weist eine Wendel mit einer Kontaktfläche auf, auf der ein mit dem Betätigungselement verbundener Spannnocken (81) gleitend gelagert ist. Der Spannnocken (81) kann auch über eine wie in Figur 2 dargestellte Spannhülse (82) mit dem Betätigungselement (40) verknüpft sein.

Bevor die Einstichbewegung ausgeführt wird, muss jeweils eine Lanzette (91) mit dem Lanzettenantrieb (30) gekoppelt werden. Dies geschieht im Rahmen der dargestellten Ausführungsform mittels eines als Stößel ausgestalteten Lanzettenhalters (2_0). An dem der Lanzette (91) zugewandten Ende des Stößels (20) ist eine verdickte Haltevorrichtung (Kopplungsgeometrie) (21) vorgesehen, die zum Ankoppeln einer Lanzette (91) in eine korrespondierende Haltevorrichtung im Haltebereich (93) des Lanzettenkörpers (91) eingeführt wird. Die Haltevorrichtung des Lanzettenkörpers (91) ist so ausgebildet, dass sie das Halteelement (21) des Stößels (20) geometrisch koppelt, wenn der Stößel (20) so weit in Einstichrichtung bewegt wird, dass sein vorderes Ende den Lanzettenkörper kontaktiert und die Lanzette (91) in Einstichrichtung verschiebt. Dadurch wird die Lanzette (91) formschlüssig an den Lanzettenantrieb (30) angekoppelt. Nähere Einzelheiten und alternative Ausführungsformen eines geeigneten Kopplungsmechanismus sind in der internationalen Patentanmeldung WO 02/36010 A1 beschrieben, deren Inhalt durch Bezugnahme zum Gegenstand der vorliegenden Anmeldung gemacht wird.

Bei der dargestellten bevorzugten Ausführungsform ist die Lanzette (91) "direkt geführt" d.h. sie befindet sich unmittelbar in einem die erforderliche Führung (92) während der

Einstichbewegung bildenden Teil des Gehäuses (10) (im vorliegenden Fall eines Magazins (90), das eine Mehrzahl von Lanzetten enthält). Die nachfolgend erläuterte erfindungsgemäße Ausführungsform des Lanzettenantriebs (30) eignet sich in besonderem Maße für solche direkt geführten magazinierten Lanzetten (91). Sie ist jedoch auch mit den bisher überwiegend gebräuchlichen indirekten Lanzettenführungen verwendbar, bei denen der Lanzettenantrieb permanent mit einem Lanzettenhalter gekoppelt ist, in den manuell für jede Blutentnahme eine neue Lanzette eingesetzt wird. Während des Einstichvorganges wird der Lanzettenhalter (20) mittels eines als Führung dienenden Gehäuse- bzw. Magazinteils (92) geführt und sorgt damit indirekt für die erforderliche Führung der Lanzette (91) auf dem Einstichweg.

Figur 3 stellt eine perspektivische Darstellung des erfindungsgemäßen Entnahmegeräts (1) aus den Figuren 1 und 2 dar, wobei zur besseren Erläuterung der Funktionsweise das Gehäuse (10) sowie das Lanzettenmagazin (90) weggelassen wurde. Das dargestellte Entnahmegerät (1) weist eine mit dem Betätigungselement (40) verbundene und als Bahnsteuerung (72, 73) ausgestaltete Arretiervorrichtung (70) auf. Die Arretiervorrichtung weist an ihrer dem Geräteinneren zugewandten Seite einen (in Figur 3 nicht sichtbaren) Steuernocken (71) auf, der entlang einer Steuerbahn (72, 73) verschiebbar gelagert ist, wobei die Steuerbahn bzw. der Steuerbahnteil (72, 73) auf dem drehbaren Antriebsrotor (50) des Drehschiebergetriebes angebracht ist. Dadurch kann der Steuernocken (71) bei Verschiebung des Betätigungselementes (40) parallel zur Gerätehauptachse (A) eine Relativbewegung bezüglich der Steuerbahn (72, 73) ausführen, wobei er den jeweiligen Abschnitt der Steuerbahn abfährt bzw. an diesem entlang gleitet. Die Steuerbahn des Steuerbahnteils weist im Rahmen dieser Ausführungsform zwei Abschnitte (72, 73) auf, wobei der erste Abschnitt (72) im Wesentlichen parallel zur Hauptgeräteachse (A), d.h. parallel zur Drehachse des Antriebsrotors (50) des Drehschiebergetriebes, verläuft. Der erste Abschnitt (72) der Steuerbahn geht unmittelbar in einen zweiten Abschnitt (73) über, wobei dieser zweite Abschnitt (73) im Wesentlichen senkrecht zur Hauptgeräteachse (A), d.h. senkrecht zur Drehachse des Antriebsrotors (50) des Drehschiebergetriebes verläuft. Die Arretiervorrichtung (70) ist gesondert nochmals in Fig. 3a dargestellt. Dabei ist das

Betätigungselement (40) transparent dargestellt, so dass der dem Antriebsrotor (50) zugewandte Steuernocken (71) sichtbar wird.

Wie insbesondere aus den Figuren 4 bis 7 ersichtlich, steht der um die Hauptgeräteachse (A) drehbare Antriebsrotor (50) unter der Wirkung des als Torsionsfeder ausgestalteten elastischen Antriebselements (31) (im Folgenden als Antriebsfeder bezeichnet). Ein Ende der Antriebsfeder (31) ist mit dem Antriebsrotor (50), das andere Ende mit dem als wendelförmigen Spannbolzen (80) ausgestalteten eingangsseitigen Getriebeglied verbunden. Die Antriebsfeder (31) wird im Rahmen dieser Ausführungsform durch eine ihrer Federkraft entgegen wirkende Drehung des wendelförmigen Spannbolzens (80) gespannt, wobei die Drehung des wendelförmigen Spannbolzens (80) durch eine Verschiebung des Betätigungselementes (40) in Richtung des proximalen Endes (2) des Entnahmesystems bewirkt wird. Durch die axiale, d.h. in Richtung des proximalen Endes des Gehäuses parallel zur Hauptgeräteachse (A) geführte Verschiebung des rotationsfest gelagerten und mit dem Betätigungselement (40) fest verbundenen Spannnockens (81), der gleitend auf der Wendel des wendelförmigen Spannbolzens (80) gelagert ist, wird eine Drehung des als wendelförmiger Spannbolzen (80) ausgestalteten eingangsseitigen Getriebegliedes und somit ein Spannen der Antriebsfeder (31) herbeigeführt.

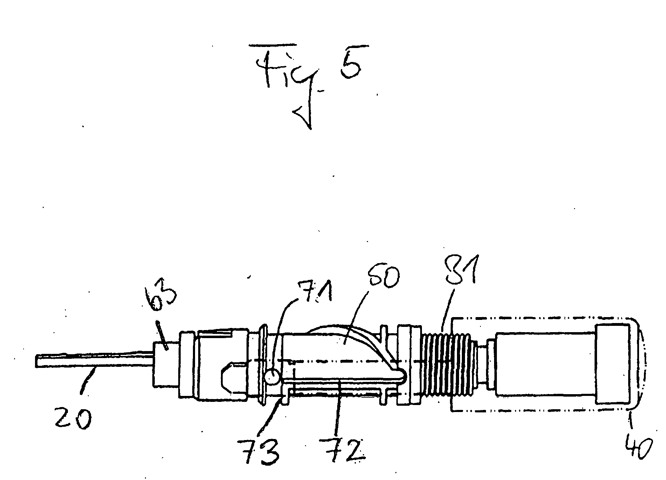

Die Figuren 4 bis 6 verdeutlichen den zeitlichen Verlauf der Funktionsweise des in den Figuren 1 bis 3 dargestellten Entnahmegerätes. Die in Zusammenhang mit den jeweils vorangegangenen Figuren dargestellten Bauteile sind dabei wie in der gesamten vorliegenden Offenbarung mit den gleichen Bezugszeichen gekennzeichnet und werden nicht nochmals beschrieben. In Figur 4 ist das erfindungsgemäße Entnahmegerät im Ausgangszustand, d.h. vor Verschiebung des Betätigungselementes (40) dargestellt. Der Spannocken befindet sich (nicht sichtbar) innerhalb der Spannhülse (82). Der Steuernocken (71) befindet sich am Anfangspunkt des ersten, parallel zur Rotationsachse des Antriebsrotors verlaufenden Abschnittes (72) des Steuerbahnteils der Bahnsteuerung. Das Betätigungselement (40) ist transparent dargestellt. Der Antriebsrotor (50) des Drehschiebergetriebes sowie der Lanzettenhalter (20) befinden sich in ihren Ausgangspositionen. Das Betätigungselement (40) wird anschließend von dem in Figur 4 dargestellten Ausgangszustand durch Verschieben in Richtung des proximalen Endes (2) des Entnahmesystems in den betätigten Zustand überführt. Dabei gleitet der in der Spannhülse (82) angeordnete und in Figur 4 nicht sichtbare Spannnocken (81) über die Wendel des wendelförmigen Spannbolzens (80), wodurch dieser gedreht und dadurch die Antriebsfeder (31) gegen den ruhenden, durch den Steuernocken (71) der Bahnsteuerung gehaltene Antriebsrotor (50) gespannt wird. Gleichzeitig fährt der über die Arretiervorrichtung (70) mit dem Betätigungselement (40) fest verbundene Steuernocken (71) den ersten, parallel zur Drehachse des Antriebsrotors verlaufenden Abschnitt (72) der Steuerbahn in Richtung des proximalen Endes (2) des Entnahmesystems ab, bis der Steuernocken (71) den Anfang des zweiten, senkrecht zur Rotationsachse des Drehschiebergetriebes bzw. Antriebsrotors verlaufenden Abschnitt (73) erreicht. Dieser Zustand, bei dem das Betätigungselement (40) maximal in Richtung des proximalen Endes (2) des Entnahmegerätes verschoben ist, ist in Figur 5 dargestellt. In diesem Zustand beginnt sich der Antriebsrotor (50) durch Einwirkung der Federkraft der gespannten Antriebsfeder (31) um seine Rotationsachse (A) zu drehen. Dabei befinden sich die Vortriebshülse (63) und der damit fest verbundene Lanzettenhalter (20) noch in der Ausgangsposition zu Beginn der Einstichbewegung.

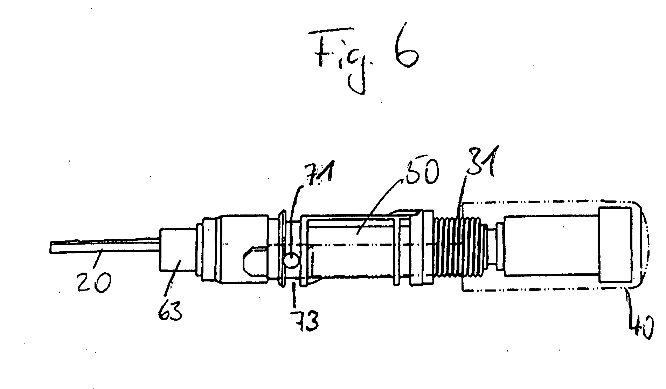

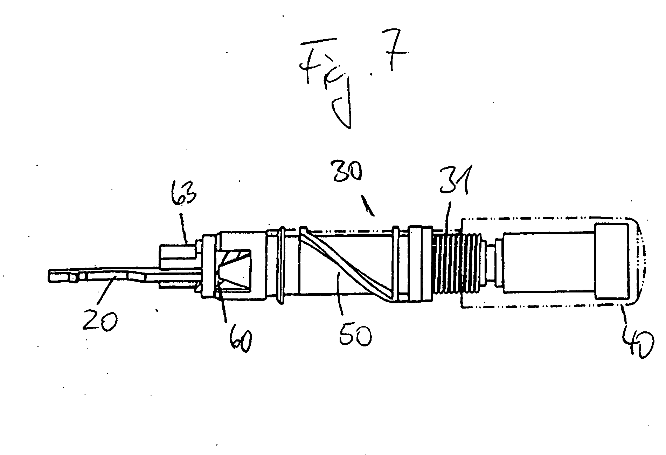

Figur 6 stellt einen zeitlich darauf folgenden Zustand des erfindungsgemäßen Entnahmegeräts dar, bei dem sich der Antriebsrotor (50) bereits um etwa 90° um seine Rotationsachse gedreht hat und der kurvengeführte Lanzettenhalter (20) bereits in einer in Einstichrichtung ausgelenkten Position befinden. Figur 7 stellt den gleichen Zustand des Entnahmegerätes dar, jedoch von der gegenüberliegenden Seite aus betrachtet. Dabei wird auch der abtriebsseitige, als Kurvensteuerung (60) ausgestaltete Kopplungsmechanismus sichtbar.

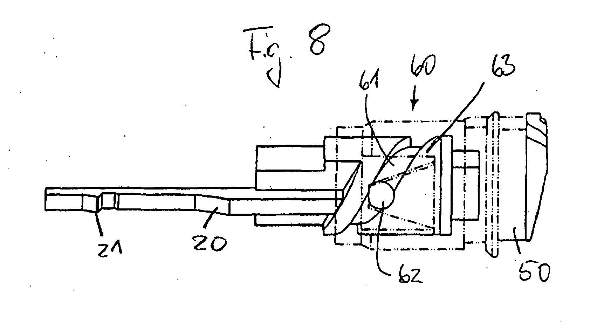

Durch einen in den Figuren 8 bis 10 dargestellten abtriebsseitigen Kopplungsmechanismus wird die Drehbewegung des Antriebsrotors (50) in die Einstechbewegung umgesetzt, die über den Lanzettenhalter (20) auf eine daran angekoppelte Lanzette (91) übertragen wird. Der abtriebsseitige Kopplungsmechanismus wird im dargestellten Fall von einer Kurvensteuerung (60) mit einer Stechkurve bzw. einem Stechprofil (61) und einem die Stechkurve bzw. das Stechprofil (61) während der Einstichbewegung abfahrenden Steuerzapfen (62) gebildet. Bei der dargestellten Ausführungsform wird die Steuerkurve (61) von einer am Umfang der Vortriebshülse (63) umlaufenden Ausnehmung gebildet. Der Steuerzapfen (62) ist am Antriebsrotor (50) ausgebildet, der von dem mit der Stechkurve bzw. dem Stechprofil (61) versehenen Teil der Vortriebshülse (63) umschlossen ist.

Die Vortriebshülse (63) ist durch eine nicht dargestellte Längsnut drehfest so geführt, dass sie nur eine Translationsbewegung ausführen kann. An ihrem vorderen Ende ist der Lanzettenhalter (20) starr fixiert.

Die Kurvensteuerung (60) funktioniert grundsätzlich in gleicher Weise, wie die in der US 5,318,584 und in der EP 1 034 740 A1 beschriebenen Kurvensteuerungen. Ein wesentlicher Unterschied besteht jedoch darin, dass der Antriebsrotor (50) während des Spannens der Antriebsfeder (31) nicht zurückgedreht werden muss. Deshalb kann einerseits eine sehr einfache Gestaltung der Stechkurve bzw. des Stechprofils (61) gewählt und andererseits der gesamte Drehwinkel von 360° für die Umsetzung der Drehbewegung des Antriebsrotors (50) in eine Translationsbewegung des Lanzettenhalters (20) und einer damit verbundenen Lanzette (91) genutzt werden.

Dies kann dadurch erreicht werden, dass die Spanneinrichtung erfindungsgemäß bevorzugt gemäß dem OWADAC-Prinzip (One Way Alternating Drive And Cocking) konstruiert ist, wie es in der EP 1 384 438 A1 offenbart ist, auf die diesbezüglich hiermit Bezug genommen wird. Das von dem Antriebsrotor (50) abgewandte Ende der Antriebsfeder (31) stützt sich dabei gegen ein als wendelförmiger Spannbolzen (80) ausgestaltetes eingangsseitiges Getriebeelement ab, welches zum Spannen der Antriebsfeder (31) bei gehemmter Rotation des Antriebsrotors (50) in die gleiche Drehrichtung drehbar ist, in die sich der Antriebsrotor (50) während der Einsstichbewegung dreht. Während der Einstichbewegung ist der wendelförmige Spannbolzen (80) gegen eine Rückwärtsdrehung arretiert, so dass der Antriebsrotor (50) nach Freigabe der seine Rotation verhindernden Arretiervorrichtung die Drehbewegung durchführt, die in die Einstichbewegung der Lanzette (91) umgesetzt wird.

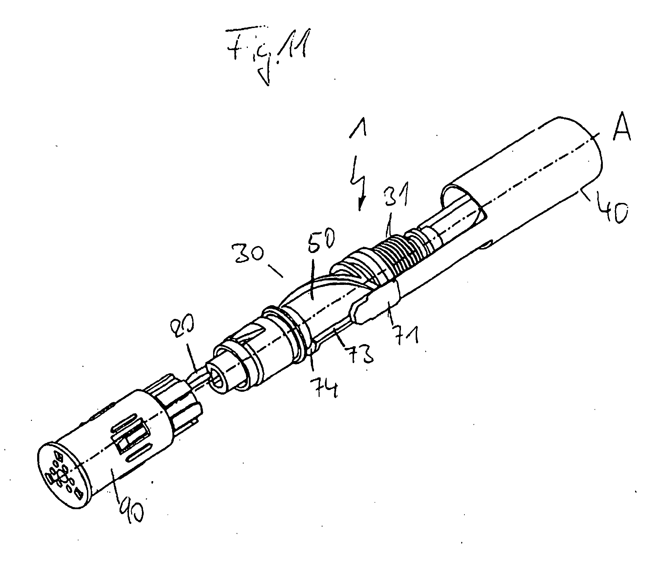

In Figur 11 ist schließlich eine perspektivische Darstellung des in Fig. 1a dargestellten Entnahmegerätes (1) mit aufgesetztem Lanzettenmagazin (90) jedoch ohne Gehäuse (10) dargestellt.

## Patentansprüche

1. System zur Entnahme von Körperflüssigkeiten für Diagnosezwecke, umfassend
- ein Entnahmegerät (1),
- ein Gehäuse (10) mit einer Austrittsöffnung für eine Lanzettenspitze,
- einen in dem Gehäuse (10) entlang eines vorbestimmten Einstichweges beweglichen Lanzettenhalter (20) zur auswechselbaren Halterung einer Lanzette (91),
- ein Magazin (90) mit einer Mehrzahl von auf das Entnahmegerät (1) abgestimmten Lanzetten (91) mit einem Lanzettenkörper und einer Lanzettenspitze, die nacheinander an den Lanzettenhalter (20) angekoppelt werden können, wobei die vorgesehenen Lanzetten mit je einem Sterilschutz (94) versehen sind, der die Sterilität von noch unbenutzten Lanzetten bis zum Einstich gewährleistet und der aus einem elastomeren Material besteht und beim Einstich durch die zu schützende Lanzettenspitze durchstochen oder abgestreift wird,
- eine Lanzettenführung (92) zur Führung des Lanzettenhalters (20) auf dem vorbestimmten Einstichweg nach Auslösen der Einstichbewegung,
- einen Lanzettenantrieb (30) mit einem elastischen Antriebselement (31), der sich durch Spannen aus einem ungespannten in einen gespannten Zustand überführen lässt und durch den nach dem Auslösen die Entspannungsbewegung des gespannten elastischen Antriebselementes (31) in eine Einstichbewegung umgesetzt wird, bei der die vom Lanzettenhalter (20) gehaltene Lanzette (91) entlang des vorbestimmten Einstichweges in Einstichrichtung bewegt wird, bis die Lanzettenspitze zumindest teilweise aus der Austrittsöffnung austritt, und durch den der Lanzettenhalter (20) in eine Position zurückgeführt wird, bei der die Spitze der Lanzette (91) sich in dem Gehäuse (10) befindet, und
- eine kombinierte Spann- und Auslöseeinrichtung mit einem von der Außenseite des Gehäuses (10) zugänglichen Betätigungselement (40), das einen Ausgangszustand und einen betätigten Zustand aufweist, wobei die kombinierte Spann- und Auslöseeinrichtung eine Arretiervorrichtung (70) aufweist, die mit dem Betätigungselement (40) und dem Lanzettenantrieb (30) derart mechanisch gekoppelt ist, dass der Lanzettenantrieb (30) bei Verschieben des Betätigungselementes (40) entlang des Betätigungsweges zunächst gespannt und dann ausgelöst wird, wobei die Auslösung des Lanzettenantriebs (30) bei Erreichen eines bestimmten Punktes entlang des Betätigungsweges freigegeben wird.

2. System nach Anspruch 1, **dadurch gekennzeichnet, dass** die Arretiervorrichtung (70) als Bahnsteuerung ausgestaltet ist, die als Glieder einen Steuerbahnteil und einen Steuernocken (71) umfasst, wobei der Steuernocken (71) während mindestens eines Teils der Verschiebung des Betätigungselementes (40) entlang des Betätigungsweges eine Relativbewegung bezüglich des Steuerbahnteils macht, bei der er die Steuerbahn des Steuerbahnteils zumindest teilweise abfährt.

3. System nach einem der vorhergehenden Ansprüche, **dadurch gekennzeichnet, dass** der Lanzettenantrieb (30) ein Drehschiebergetriebe mit einem drehbaren Antriebsrotor (50) aufweist, durch welchen ein auf der Eingangsseite des Drehschiebergetriebes eingeleitetes Drehmoment in eine Längsverschiebung in Richtung des vorbestimmten Einstichweges umgewandelt wird, wobei der Antriebsrotor (50) mit dem elastischen Antriebselement (31) gekoppelt ist und die ausgangsseitige Längsverschiebung des Drehschiebergetriebes auf den Lanzettenhalter (20) übertragen wird.

4. System nach Anspruch 2, **dadurch gekennzeichnet, dass** der Steuerbahnteil der Bahnsteuerung zumindest teilweise auf dem Antriebsrotor (50) angebracht ist.

5. System nach einem der Ansprüche 2 oder 4, **dadurch gekennzeichnet, dass** der Steuerbahnteil einen ersten (72) und einen zweiten Abschnitt (73) umfasst, wobei der erste Abschnitt des Steuerbahnteils (72) im Wesentlichen parallel zur Drehachse des Drehschiebergetriebes verläuft und wobei der zweite Abschnitt des Steuerbahnteils (73) im Wesentlichen senkrecht zur Drehachse des Drehschiebergetriebes verläuft.

6. System nach einem der vorhergehenden Ansprüche, **dadurch gekennzeichnet, dass** das elastische Antriebselement des Lanzettenantriebs durch stetiges, in eine Richtung gerichtetes Verschieben des Betätigungselementes zunächst aus einem ungespannten in einen gespannten Zustand überführt Wird und anschließend ausgelöst wird.

7. System nach einem der vorhergehenden Ansprüche, **dadurch gekennzeichnet, dass** das Betätigungselement (40) ein aus dem hinteren, von der Austrittöffnung für die Lanzettenspitze abgewandten Ende des Gehäuses (10) herausragender Bedienknopf ist.

8. System nach einem der Ansprüche 3 bis 7, **dadurch gekennzeichnet, dass** das Drehschiebergetriebe eine Kurvensteuerung (60) mit einer eine Steuerkurve (61) bildenden, zusammen mit dem Antriebsrotor (50) drehbaren Ausnehmung aufweist, in die ein passender Steuerzapfen (62) eingreift, wobei mindestens ein Teil der Einstich- und Rückführbewegung durch eine Relativbewegung zwischen dem Steuerzapfen (62) und der Ausnehmung bestimmt wird, bei der der Steuerzapfen (62) die durch die Ausnehmung gebildete Steuerkurve abfährt.

9. System nach einem der Ansprüche 3 bis 8, **dadurch gekennzeichnet**, das die Eingangsseite des Drehschiebergetriebes mittels einer an dem drehbaren Getriebeglied vorgesehenen Wendel und mittels eines mit dem Betätigungselement (40) verbundenen, längs des Betätigungsweges (40) beweglichen und auf einer Gleitfläche der Wendel mittels einer Kontaktfläche gleitbaren Spannnockens (81) ausgebildet ist.

10. System nach einem der vorhergehenden Ansprüche, **dadurch gekennzeichnet, dass** das Magazin (90) zumindest einen Teil der Lanzettenführung (92) umfasst.

11. System nach einem der vorhergehenden Ansprüche, **dadurch gekennzeichnet, dass** die Lanzette (91) während des gesamten Einstichvorganges in mechanischer Kopplung mit dem Lanzettenantrieb (30) steht.

12. System nach einem der vorhergehenden Ansprüche, **dadurch gekennzeichnet, dass** es Mittel zur Anzeige des bevorstehenden Auslösens der Einstichbewegung umfasst.

13. System nach Anspruch 12, **dadurch gekennzeichnet, dass** die Mittel zur Anzeige des bevorstehenden Auslösens der Einstichbewegung Teil des Bedienelementes sind.

## Claims

1. System for withdrawing body fluids for diagnostic purposes, comprising
- a withdrawal appliance (1),
- a housing (10) having an exit opening for a lancet tip,
- a lancet holder (20), which can be moved along a predetermined puncture path in the housing (10), for replaceably holding a lancet (91),
- a magazine (90) having a plurality of lancets (91) which are tailored to the withdrawal appliance (1) and have a lancet body and a lancet tip and can be coupled in succession to the lancet holder (20), wherein the lancets provided are each provided with a sterile protection (94), which ensures the sterility of as yet unused lancets prior to puncturing and which consists of an elastomer material and is pierced or stripped off by the lancet tip, which is to be protected, during puncturing,
- a lancet guide (92) for guiding the lancet holder (20) on the predetermined puncture path after the puncturing movement has been triggered,
- a lancet drive (30) having an elastic drive element (31) which can be changed by tensioning from an untensioned to a tensioned state and by means of which, after triggering, the relaxation movement of the tensioned elastic drive element (31) is converted into a puncturing movement, in the course of which the lancet (91) held by the lancet holder (20) is moved along the predetermined puncture path in a puncturing direction until the lancet tip emerges at least partially from the exit opening, and by means of which the lancet holder (20) is returned to a position in which the tip of the lancet (91) is located in the housing (10), and
- a combined tensioning and triggering device having an actuating element (40) which is accessible from the outside of the housing (10) and has an initial state and an actuated state, wherein the combined tensioning and triggering device has a locking apparatus (70), which is mechanically coupled to the actuating element (40) and the lancet drive (30) in such a manner that the lancet drive (30) is firstly tensioned and then triggered when the actuating element (40) is displaced along the actuation path, the triggering of the lancet drive (30) being enabled when a certain point along the actuation path is reached.

2. System according to Claim 1, **characterized in that** the locking apparatus (70) is configured as a track control unit comprising a control track part and a control cam (71) as members, wherein the control cam (71) performs a relative movement with respect to the control track part during at least part of the displacement of the actuating element (40) along the actuation path, in the course of which it travels along at least part of the control track of the control track part.

3. System according to either of the preceding claims, **characterized in that** the lancet drive (30) has a rotary slide transmission having a rotatable drive rotor (50), by means of which a torque induced on the input side of the rotary slide transmission is converted into a longitudinal displacement in the direction of the predetermined puncture path, wherein the drive rotor (50) is coupled to the elastic drive element (31) and the output-side longitudinal displacement of the rotary slide transmission is transferred to the lancet holder (20).

4. System according to Claim 2, **characterized in that** the control track part of the track control unit is mounted at least partially on the drive rotor (50).

5. System according to either of Claims 2 and 4, **characterized in that** the control track part comprises a first portion (72) and a second portion (73), wherein the first portion of the control track part (72) runs substantially parallel to the axis of rotation of the rotary slide transmission, and wherein the second portion of the control track part (73) runs substantially perpendicular to the axis of rotation of the rotary slide transmission.

6. System according to one of the preceding claims, **characterized in that** the elastic drive element of the lancet drive is firstly changed from an untensioned to a tensioned state by continuous unidirectional displacement of the actuating element and is then triggered.

7. System according to one of the preceding claims, **characterized in that** the actuating element (40) is an operating button protruding out of the rear end of the housing (10) remote from the exit opening for the lancet tip.

8. System according to one of Claims 3 to 7, **characterized in that** the rotary slide transmission has a cam control unit (60) having a recess which forms a guide curve (61), is rotatable together with the drive rotor (50) and into which a matching guide pin (62) engages, wherein at least part of the puncturing and return movement is determined by a relative movement between the guide pin (62) and the recess, in the course of which the guide pin (62) travels along the guide curve formed by the recess.

9. System according to one of Claims 3 to 8, **characterized in that** the input side of the rotary slide transmission is formed by means of a helix provided on the rotatable transmission member and by means of a tensioning cam (81) which is connected to the actuating element (40), can move along the actuation path (40) and can slide on a sliding surface of the helix by means of a contact surface.

10. System according to one of the preceding claims, **characterized in that** the magazine (90) comprises at least part of the lancet guide (92).

11. System according to one of the preceding claims, **characterized in that** the lancet (91) is mechanically coupled to the lancet drive (30) throughout the entire puncturing operation.

12. System according to one of the preceding claims, **characterized in that** it comprises means for indicating the imminent triggering of the puncturing movement.

13. System according to Claim 12, **characterized in that** the means for indicating the imminent triggering of the puncturing movement are part of the operating element.

## Revendications

1. Système de prélèvement de fluides corporels à des fins de diagnostic, comprenant
- un instrument de prélèvement (1),
- un logement (10) avec une ouverture de sortie pour une pointe de lancette,
- un support de lancette (20) mobile dans le logement (10) le long d'un trajet de piqûre prédéterminé pour la fixation amovible d'une lancette (91),
- un réservoir (90) avec une pluralité de lancettes (91) adaptées à l'instrument de prélèvement (1) ayant un corps de lancette et une pointe de lancette, qui peuvent être couplés tour à tour au support de lancette (20), les lancettes prévues étant dotées chacune d'une protection stérile (94), qui garantit jusqu'à la piqûre la stérilité des lancettes pas encore utilisées et qui est constituée d'un matériau élastomère et qui, lors de la piqûre, est perforée ou enlevée par le biais de la pointe de lancette à protéger,
- un guidage de lancette (92) pour le guidage du support de lancette (20) sur le trajet de piqûre prédéterminé après le déclenchement du mouvement de piqûre,
- un entraînement de lancette (30) avec un élément d'entraînement élastique (31), qui passe d'un état non tendu à un état tendu par le biais d'une tension et par le biais duquel après le déclenchement le mouvement de piqûre de l'élément d'entraînement élastique (31) tendu se transforme en un mouvement de piqûre, lors duquel la lancette (91) retenue par le support de lancette (20) est déplacée le long du trajet de piqûre prédéterminé dans la direction de piqûre, jusqu'à ce que la pointe de lancette sorte au moins partiellement de l'ouverture de sortie, et par le biais duquel le support de lancette (20) est ramené dans une position dans laquelle la pointe de la lancette (91) se trouve dans le logement (10), et
- un dispositif combiné de tension et de déclenchement avec un élément d'actionnement (40) accessible par la face externe du logement (10), lequel élément présente un état initial et un état activé, le dispositif combiné de tension et de déclenchement présentant un dispositif de verrouillage (70) qui est couplé mécaniquement à l'élément d'actionnement (40) et à l'entraînement de lancette (30) de telle sorte que l'entraînement de lancette (30) est tout d'abord tendu lors du déplacement de l'élément d'actionnement (40) le long du trajet de piqûre puis déclenché, le déclenchement de l'entraînement de lancette (30) étant initié lorsqu'un certain point le long du trajet d'actionnement est atteint.

2. Système selon la revendication 1, **caractérisé en ce que** le dispositif de verrouillage (70) est conçu en tant que commande continue, qui comprend en tant qu'organes une partie à trajectoire de commande et une came de commande (71), la came de commande (71) pendant au moins une partie du déplacement de l'élément d'actionnement (40) le long du trajet d'actionnement effectuant un mouvement relatif par rapport à la partie à trajectoire de commande, lors duquel elle emmène au moins partiellement la trajectoire de commande de la partie à trajectoire de commande.

3. Système selon l'une des revendications précédentes, **caractérisé en ce que** l'entraînement de lancette (30) présente un mécanisme de transmission avec un rotor d'entraînement rotatif (50), par le biais duquel un couple de rotation introduit au niveau du côté entrée du mécanisme de transmission est transformé en un déplacement longitudinal en direction du trajet de piqûre prédéterminé, le rotor d'entraînement (50) étant couplé à l'élément d'entraînement élastique (31) et le déplacement longitudinal côté sortie du mécanisme de transmission étant transmis au support de lancette (20).

4. Système selon la revendication 2, **caractérisé en ce que** la partie à trajectoire de commande de la commande continue est appliquée au moins partiellement sur le rotor d'entraînement (50).

5. Système selon l'une des revendications 2 ou 4, **caractérisé en ce que** la partie à trajectoire de commande comprend une première (72) et une deuxième section (73), la première section de la partie à trajectoire de commande (72) étant pour l'essentiel parallèle à l'axe de rotation du mécanisme de transmission et la deuxième section de la partie à trajectoire de commande (73) étant pour l'essentiel perpendiculaire à l'axe de rotation du mécanisme de transmission.

6. Système selon l'une des revendications précédentes, **caractérisé en ce que** l'élément d'entraînement élastique de l'entraînement de lancette passe tout d'abord d'un état non tendu à un état tendu par le biais d'un déplacement de l'élément d'actionnement continu et orienté dans une direction et est ensuite déclenché.

7. Système selon l'une des revendications précédentes, **caractérisé en ce que** l'élément d'actionnement (40) est un bouton d'actionnement faisant saillie de l'extrémité arrière du logement (10) opposée à l'ouverture de sortie pour la pointe de lancette.

8. Système selon l'une des revendications 3 à 7, **caractérisé en ce que** le mécanisme de transmission présente une commande à came (60) avec un évidement formant une came de commande (61) et apte à tourner avec le rotor d'entraînement (50), évidement dans lequel un tourillon de commande ajusté (62) vient s'insérer, au moins une partie du mouvement de piqûre et de retour étant déterminé par le biais d'un mouvement relatif entre le tourillon de commande (62) et l'évidement, mouvement lors duquel le tourillon de commande (62) emmène la came de commande formée par le biais dudit évidement.

9. Système selon l'une des revendications 3 à 8, **caractérisé en ce que** le côté entrée du mécanisme de transmission est réalisé au moyen d'une hélice prévue au niveau de l'organe de transmission rotatif et au moyen d'une came de tension (81) reliée à l'élément d'actionnement (40), mobile le long du trajet d'actionnement (40) et apte à glisser sur une surface de glissement de l'hélice au moyen d'une surface de contact.

10. Système selon l'une des revendications précédentes, **caractérisé en ce que** le réservoir (90) comprend au moins une partie du guidage de lancette (92).

11. Système selon l'une des revendications précédentes, **caractérisé en ce que** la lancette (91) est couplée mécaniquement à l'entraînement de lancette (30) pendant la totalité du processus de piqûre.

12. Système selon l'une des revendications précédentes, **caractérisé en ce qu'**il comprend des moyens pour indiquer le déclenchement imminent du mouvement de piqûre.

13. Système selon la revendication 12, **caractérisé en ce que** les moyens pour indiquer le déclenchement imminent du mouvement de piqûre font partie de l'élément d'actionnement.
